# EUROPEAN PATENT APPLICATION

(11) **EP 1 950 304 A1**
(43) Date of publication of application: **30.07.2008**
(21) Application number: 07101268.6
(22) Date of filing: 26.01.2007
(51) Int. Cl.: C12N 15/82

(54) **Identification, classification and optionally stacking of r-genes in solanum using an effector-receptor approach**

(71) Applicant: Coöperatie AVEBE U.A., 9641 GK Veendam (NL)
(72) Inventor: Jacobsen, Evert, 6706 BD Wageningen (NL); Visser, Richard Gerardus Franciscus, 6721 ET Bennekom (NL); van der Vossen, Edwin Andries Gerard, 3572 ZM Utrecht (NL); Vleeshouer, Vivianne, 9641 GK Veendam (NL)
(74) Representative: van Loon, C.J.J.

(57) **Abstract**

The invention relates to the field of resistance genes, more in particular to resistance genes from potato plants. The invention particularly relates to a method for identifying a resistance gene (an R-gene) comprising using a set of defined Phytophthora effectors. The invention describes an approach that is based on the use of a particular Phytophthora effector (preferably an elicitor) and linking said effector with a resistance gene.

## Description

The invention relates to the field of resistance genes, more in particular to resistance genes from potato plants. The invention particularly relates to a method for identifying a resistance gene (an R-gene) comprising using a set of defined Phytophthora effectors.

The oomycete *Phytophthora infestans* causes late blight, the devastating potato disease that lead to Irish famines in 1840s. Late blight is mainly controlled by application of enormous amounts of chemicals, and to limit chemical control there is an urgent need for resistant potato cultivars. Recent *Solanum* germplasm surveys suggest a wealth of resistance (R) genes that can be exploited in the future to develop resistant varieties. In contrast to introgression breeding, isolation of such genes from *Solanum* species and their stable transformation into existing potato or tomato varieties is by far the fastest means of exploiting potentially durable late blight resistance present in the *Solanum* gene pool. The present inventors have an extensive collection of wild *Solanum* germplasm present in vitro. This set is the result of a broad screening of 5,000 genotypes belonging to *Solanum* sect. Petota (tuber-bearing *Solanum).* After monitoring the resistance in vitro, leaf tests and field trials, with various *P. infestans* isolates, the present collection comprises more than 500 genotypes, which belong to more than 145 different species. Large numbers of crosses have been made, and populations segregating for late blight resistance are being identified.

Wild *Solanum* species have previously been subject to mapping of genes for resistance to *P. infestans.* A summary of mapped R-genes for late blight resistance is given in Table **1.** In early breeding programs, eleven *R* genes (R1-R11) from *S*. *demissum* were introduced into cultivated potato. Recent work has shown that most of these genes, i.e. R3, R5-R11 localize on chromosome 11. Despite the conserved genetic position of these *R* genes on the genome, functional diversity between them is evident. *R3* resistance to *P. infestans* is for example conferred by two closely linked *R* genes, *R3a* and *R3b,* with distinct specificities (Huang et al., MPMI 17: 428-435, 2004). Recently, comparative genomics enabled the isolation of the *R3a* late blight resistance gene in potato (Huang et al., Plant Journal 42: 251-261, 2005). In addition to *R3a,* three other R-genes for late blight resistance have been cloned and all, like *R3a,* belong to the NB-LRR class of plant R-genes; *R1* from *S. demissum* (Ballvora et al., Plant Journal 30: 361-371, 2002) and *Rpi-blb1* and *Rpi-blb2* from *S*. *bulbocastanum* (van der Vossen et al, Plant Journal 36: 867-882, 2003 & van der Vossen et al., Plant Journal 44: 208-222, 2005).

### Effector based resistance approach

The resistance or susceptibility response of *Solanum* to *P. infestans* is determined by the interaction of plant proteins with secreted effector proteins of *P. infestans.* Specific effector molecules can be detected by R proteins, resulting in the activation of the hypersensitive response (HR). In such cases, the effector is termed an avirulence (Avr) protein. Recently, four oomycete *Avr* genes were identified: *Avr 1b-1* from the soybean pathogen *Phytophthora sojae, ATR13* and *ATR1^{NdWsB}* from the *Arabidopsis* pathogen *Hyaloperonospora parasitica* and *Avr3α* from *P. infestans* ATR13, ATR1^{NdWsB} and AVR3a are detected by cognate R proteins in the host cytoplasm, which is consistent with the hypothesis that oomycetes deliver effectors to the inside of host cells, potentially through haustoria. Sequence alignment of oomycete AVR proteins revealed a conserved motif (RXLR) within 32 amino acids of the predicted signal peptides (Rehmany et al., Plant Cell 17: 1839-1850, 2005). The RXLR motif is similar to the host-cell-targeting signal RXLX(E/Q), which is required for the translocation of proteins from malaria parasites (*Plasmodium* species) into host erythrocytes. This suggests that RXLR functions as a signal that mediates trafficking of AVR proteins into host cells. Indeed, it has been demonstrated that the RXLR motif of *P. infestans* can deliver the green fluorescent protein (GFP) from *Plasmodium* inside the red blood cell. Bioinformatic analyses indicate that the RXLR motif is common in *P*. *infestans, P. sojae* and *P*. *ramorum* and that it occurs in about 100 proteins predicted to be secreted (Pex, *Phytophthora* extracellular proteins). The laboratory of Sophien Kamoun at The Ohio State University has already identified about 50 families of genes containing the RXLR motif in *P. infestans.* One of the first families in this RXLR-Pex set contains Avr3a, the cognate AVR protein of *R3a,* and this proves the usefulness of this collection is for the identification of candidate AVR genes.
In this application we demonstrate how novel *R* genes can be identified and cloned using these RXLR proteins in a functional assay. A unique opportunity of this approach is that identified *R* genes can be classified based on the type of cognate effectors, and that the functional response provides insight into the working spectra of the novel *R* gene (see Table 5 for explanation and Table 6 for an example).

### Classification of R genes

In the past, single *R* genes were quickly overcome after introgression in potato, yet for the future multiple *R* genes need to be combined simultaneously. Since extensive resistance screenings in our laboratory are providing a continuous inflow of novel *R* genes from a diversity of *Solanum* species, we expect to have a collection of *R* genes to choose from in the near future. The challenge now is to prioritize which *R* gene should be cloned, and also, which combinations of R genes should be made for application. The main criteria are to achieve a broad spectrum of resistance (acting against many isolates), a high level of the resistance (combining two different weak *R* genes can still achieve satisfactory level in the field), and enhanced durability (combination of *R* genes interacting with *different* effectors may be less easy to break). To select the best *R* genes for application, available candidates should be classified. Up till now, *R* genes can only be classified based on the donor species and their genetic localization (at least before cloning). Here we disclose new ways for classification, mainly based on functionality. *R* genes can be categorized based on the effector they interact with (Tables 5 and 6). Based on the response pattern of an R gene to a set of effectors, one can avoid combining R genes that have the same effector recognition capacity and thus choose and combine R genes that interact with diverse effectors (Table 5). For example, we expect that a combination of different *R* genes responding to the same (set of) effector(s) will not broaden the resistance spectrum, and most likely not enhance the level of resistance or durability. Another application on the longer term may be to make the optimal selection of R genes feasible by monitoring the *P. infestans* population, for each area where the potatoes are grown.

In a first embodiment, the invention provides a method for identifying a resistance gene (R-gene) in Solanum comprising
providing a set of defined Phytophthora effectors,
exposing a part of a Solanum plant to said set of defined effectors,
identifying the presence or absence of a reaction of said plant to an effector from said set of defined effectors,
transferring at least part of the nucleic acid sequence of said Solanum to another plant
and testing the obtained plant or progeny thereof for an acquired reaction against at least one effector from said set of defined effectors.

The identification of an R-gene effectively results in a determination of the presence of an R-gene, i.e. the invention also provides a method for determining the presence of an R-gene. Preferably, the used Solanum is a wild type Solanum, i.e. a Solanum that is naturally present anywhere in the world or in a different wording a Solanum that has not been changed by genetic modification processes. Even more preferred a Solanum is used which is resistant against one or preferably multiple Phytophtora strains/isolates (the term are used interchangeable herein). Table 1 provides examples of such a Solanum.

An R-gene is typically described as a gene whose product is capable to provide at least partial resistance against a disease, more preferably a gene product capable of providing at least partial resistance to Phytophthora, even more preferably to Phytophthora infestans, the causal agent of light blight. Currently 4 R-genes for late blight resistance have been cloned and the present invention provides a method to obtain further R-genes which may be but are not necessarily homologues of already cloned R-genes.

The term effector is typically used to describe a pathogen molecule that manipulates host cell structure and function and thereby facilitating infection and/or triggering defense responses. An effector can be an elicitor and/or a toxin.

Effector genes can for example be identified as described by Kamoun (Annu. Rev. Phytopathology 44: 41-60, 2006):
"Biochemical, genetic, and bioinformatic strategies, often in combinations, have been applied to the identification of effector genes from oomycetes. Traditionally, effectors have been identified by biochemical purification and genetic analyses. With the advent of genomics, novel strategies have emerged. The implementation of functional genomics pipelines to identify effectors from sequence data sets has proved particularly successful. A typical pipeline consists of two major steps: use of data mining tools to identify candidate genes that fulfil a list of specific criteria, followed by analysis and validation of these candidate genes by functional assays, such as expression in planta and evaluation for effector-like activities.
A critical milestone in the identification of oomycete effector genes was the validation of the concept that effector proteins must be secreted in order to reach their cellular targets at the intercellular interface between the plant and pathogen or inside the host cell. Identification of candidate secreted proteins was facilitated by the fact that in oomycetes as in other eukaryotes, most secreted proteins are exported through the general secretory pathway via short, N-terminal, amino-acid sequences known as signal peptides. Signal peptides are highly degenerate and cannot be identified using DNA hybridization or PCR-based techniques. Nonetheless, computational tools, particularly the SignalP program that was developed using machine learning methods (Nielson et al, Protein Eng. 12: 3-9, 1999), can assign signal peptide prediction scores and cleavage sites to unknown amino acid sequences with a high degree of accuracy. Therefore, with the accumulation of oomycete cDNA and genome sequences, lists of candidate secreted proteins could be readily generated using bioinformatics tools. For instance, Torto et al. (Genome Res. 13: 1675-1685, 2003) developed PexFinder (with Pex standing for *Phytophthora* extracellular protein), an algorithm based on SignalP v2.0 (Nielson et al, Protein Eng. 12: 3-9, 1999) to identify proteins containing putative signal peptides from expressed sequence tags (ESTs). PexFinder was then applied to ESTs in *P. infestans.* Proteomic identification of secreted proteins collected from culture filtrates of *P*. *infestans* matched PexFinder predictions, convincingly validating the algorithm performance. Computational signal peptide predictions help to identify secreted proteins with no significant matches to known sequences. Annotation of cDNA and gene sequences is routinely done by similarity searches to sequences in public databases. However, this approach is limited in that it identifies only proteins with known functions and thus a large number of proteins with unknown functions are ignored. Computational predictions single out unknown secreted proteins that might otherwise be ignored. This results in a manageable list of candidates that can be further processed by sequence annotation and functional analyses.
Building up an annotated collection of computationally predicted candidate proteins that feed into a functional analysis pipeline is by no means a one-directional process. Rather, criteria for annotating and prioritizing candidate genes are continuously refined as new findings emerge and novel sequence-to-function links are established. One illustrative example is the finding that all known avirulence proteins of oomycetes carry a conserved motif (RXLR) closely following a signal peptide (see below for more details) (Rehmany et al.,Plant Cell 17: 1839-1850). Straightforward sequence pattern search tools can thus be used to discover an additional number of candidate effectors of the RXLR family from several *Phytophthora* species.
Identification of genes under diversifying selection (especially when coupled with selection criteria such as presence of secretory signals and in planta expression) can also help to select candidate effector genes (Liu et al., Mol. Biol. Evol. 22: 659-672). Based on the "arms race" model, adaptation and counteradaptation between pathogen and host is likely to drive their antagonistic coevolution and generate the evolutionary forces that shape effectors and their host targets. For instance, diversifying selection (also known as positive selection) can be an indicator of functionally important loci that contribute to the fitness of the organism. Indeed, some oomycete effector genes, e.g., *ATR13 and scr74,* exhibit extreme levels of amino acid polymorphism with evidence of diversifying selection. In the future, evolutionary analyses will have an even larger role in the identification of effector genes as genome sequences from additional oomycete species become available."

Using the above described strategy Kamoun and co-workers have currently identified about 50 families of genes containing the RXLR motif in *P*. *infestans* This subset of Phytophthora extracellular proteins harbouring the RXLR motif are called RD proteins and this set is constantly being updated and enlarged as criteria for annotating and prioritizing candidate genes are continuously refined. Examples of RD families that harbour functional Avr genes are RD6 (IPI-O) and RD7 (Avr3a).

Screening with the "defined Phytophthora effectors/"Phytophthora RD effectors" (the expressions are used interchangeably herein) is thus different from screening with a Phytophthora isolate which produces multiple (for the larger part unknown) effectors. Preferably, the RD effectors are tested by functional expression screenings of cDNAs expressing said effectors in Nicotiana or Solanum using an A. tumefaciens binary potato virus X (PVX)-based vector. Even more preferably, the vectors harbor different alleles of IPI-O.

Phytophthora RD effectors are used to inoculate a part of a Solanum plant. It is clear to the skilled person that preferably a susceptible part of said plant is inoculated and hence in a preferred embodiment, a leaf of said Solanum plant is used for inoculation.

After inoculation, the plants or leaves are incubated to allow the development of a reaction between the used effectors and the possible R-genes of said plant. After a sufficient amount of time (typically between 7 to 21 days), the plants or leaves are examined for the presence or absence of a reaction of said plant or leaf to an effector from said set of defined effectors. In a preferred embodiment, said reaction is a hypersensitive response (HR). Preferably, a selection is made for plants or leaves that show a response at more than 50% of the inoculation sites.

After selecting a plant that responds to an RD effector, at least part of the nucleic acid sequence of said selected plant (Solanum) is transferred to another plant (preferably being an at least partly Phytophthora susceptible potato or tomato).

As described above, the used effector can be a toxin or an elicitor. In a preferred embodiment said effector is an elicitor. An elicitor is typically a pathogen molecule that triggers defense responses resulting in enhanced resistance to an invading pathogen. Examples of elicitors are ATR1, ATR13, Avr1b, AVR3a and IPI-O.

In a preferred embodiment, the term "transferring" comprises crossing said Solanum plant with another Solanum plant and in yet another preferred embodiment, said transferring is preceded by identifying said R-gene and by isolating said gene from said Solanum plant and the isolated gene is subsequently transferred to another plant, preferably a Solanum and even more preferably a different variety. In yet another preferred embodiment, the plant that is used for crossing or the plant that receives the isolated gene is a plant that is at least partly susceptible to Phytophthora in general or Phytophthora infestans in specific.

As a next step the obtained plant or progeny thereof is tested for an acquired reaction against at least one effector from said set of defined effectors. This testing is for example accomplished by examination for resistance (inoculation) or by determining the response to a particular effector (agroinfection). The term "acquired" is used herein to describe a situation that, if compared to the situation before transferring, results in an improved or broader resistance due to the presence of the identified or identified and isolated R-gene.

The herein described approach is thus based on the use of a particular Phytophthora effector (preferably an elicitor) and linking said effector with a resistance gene. In other words, the herein described approach is based on a functional analysis.

In yet another preferred embodiment, the invention provides a method for identifying a resistance gene (R-gene) in Solanum comprising providing a set of defined Phytophthora effectors, exposing a part of a Solanum plant to said set of defined effectors, identifying the presence or absence of a reaction of said plant to an effector from said set of defined effectors, transferring at least part of the nucleic acid sequence of said Solanum to another plant and testing the obtained plant or progeny thereof for an acquired reaction against at least one effector from said set of defined effectors, wherein said acquired reaction is an acquired resistance for a Phytophthora isolate comprising at least one effector from said set of defined effectors. In this case a Phytophthora isolate is used instead of a defined effector to test whether resistance is acquired.

As described above, the herein described approach is thus based on the use of a particular Phytophthora effector (preferably an elicitor) and linking said effector with a resistance gene. This effector-based strategy is preferably combined with resistance assessment/disease testing using a Phytophthora strain that expresses said effector, hence confirming that the recognition of an elicitor by an R-gene has a true relation with resistance against a specific Phytophthora isolate that expresses said effector.

The herein provided method can also be used to identify functional homologues of an already known resistance gene. For the identification of a homologue of an already (partly) known resistance gene, the herein described method is further complemented by using a PCR-based allele mining strategy (described in more detail in the experimental part herein).

The invention therefore also provides a method for identifying a homologue of a (partially) known resistance gene, comprising
providing a set of defined Phytophthora effectors,
exposing a part of a Solanum plant to said set of defined effectors,
identifying the presence or absence of a reaction of said plant to an effector from said set of defined effectors,
transferring at least part of the nucleic acid sequence of said Solanum to another plant
and testing the obtained plant or progeny thereof for an acquired reaction against at least one effector from said set of defined effectors, said method further comprising
testing said obtained plant or progeny thereof for the presence of a homologue of an already known resistance gene, such as R1, R3a, Rpi-blb1 or Rpi-blb2.

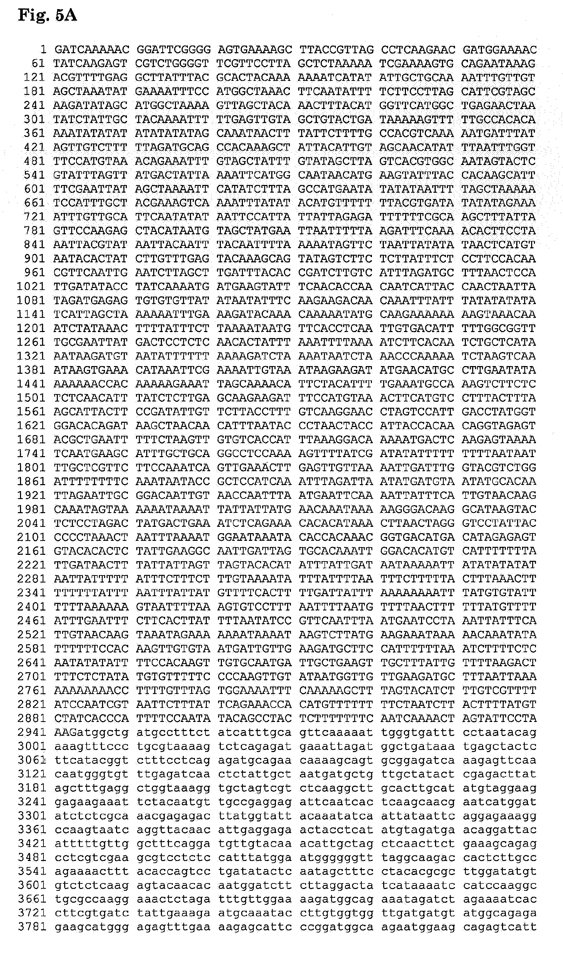

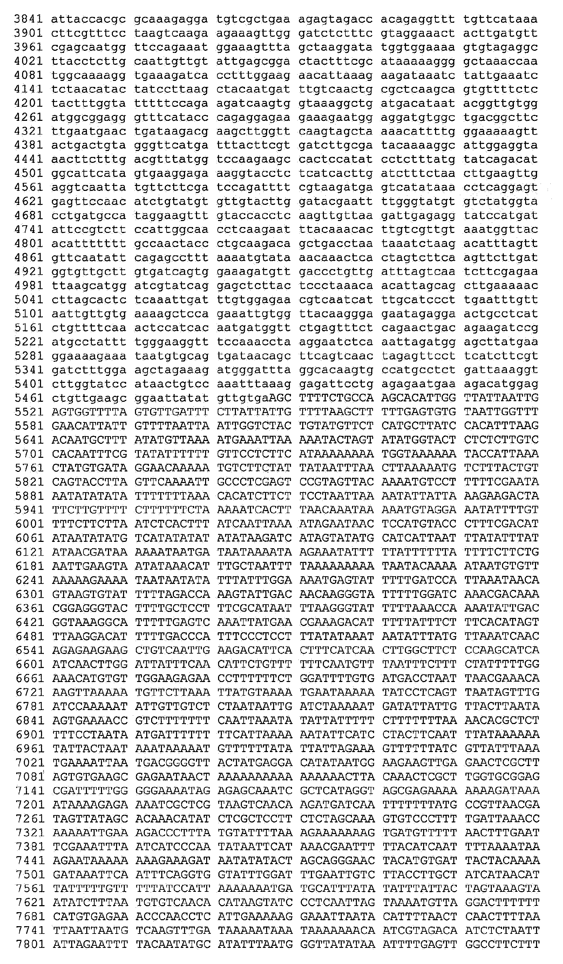

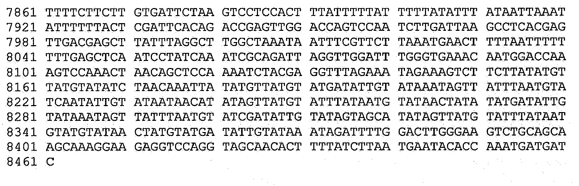

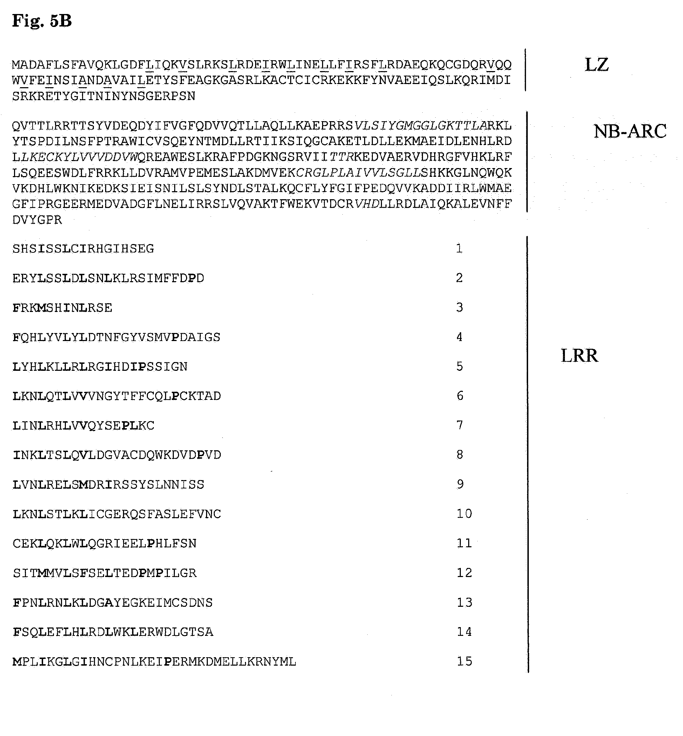

One of our co-pending applications describes the isolation of Rpi-blb3, i.e. a third resistance gene isolated from S. bulbocastanum. The nucleic acid and amino acid sequence of Rpi-blb3 is disclosed in Figure 5. Hence, also a homologue of Rpi-blb3 can be isolated by the herein described method.

The testing for the presence of a homologue is for example accomplished by performing a PCR with primers designed on the sequence of for example R1, R3a, Rpi-blb1, Rpi-blb2 or Rpi-blb3.

As an example the experimental part describes the isolation of 2 homologues of Rpi-blb1.

It is clear for the skilled person that this method can also be used to identify homologues of resistance genes which will be cloned and/or sequenced after the filing of this application.

The above disclosed methods can further be expanded by performing disease resistance assays and hence said methods optionally comprise exposing a part of said Solanum plant (or progeny thereof) or the obtained plant or progeny thereof to a Phytophthora isolate. If for example the response to effector A is linked to the presence of a resistance gene in Solanum Y, Solanum Y or progeny thereof, are preferably also tested with a Phytophthora strain expressing said effector A. Such disease resistance assays can also be applied to the plant or progeny thereof obtained from the transfer of nucleic acid from said Solanum Y to another plant. The invention therefore provides in a preferred embodiment, a method for identifying a resistance gene (R-gene) in Solanum comprising providing a set of defined Phytophthora effectors, exposing a part of a Solanum plant to said set of defined effectors,
identifying the presence or absence of a reaction of said plant to an effector from said set of defined effectors,
transferring at least part of the nucleic acid sequence of said Solanum to another plant
and testing the obtained plant or progeny thereof for an acquired reaction against at least one effector from said set of defined effectors,
, said method further comprising exposing a part of said Solanum plant to a Phytophthora isolate. In such a method the acquired reaction against at least one effector is linked to a resistance response to Phytophthora.

In yet another preferred embodiment, the invention provides a method for identifying (and optionally isolating) a resistance gene (R-gene) in (from) Solanum comprising
providing a set of defined Phytophthora effectors,
exposing a part of a Solanum plant to said set of defined effectors,
identifying the presence or absence of a reaction of said plant to an effector from said set of defined effectors,
transferring at least part of the nucleic acid sequence of said Solanum to another plant
and testing the obtained plant or progeny thereof for an acquired reaction against at least one effector from said set of defined effectors,
wherein said transferring comprises crossing said Solanum plant with another Solanum plant and optionally further comprising isolating a resistance gene from the resultant plant of the crossing or progeny thereof.

One of the above disclosed embodiments describes that the R-gene can be identified and isolated. This is for example accomplished by using molecular biology techniques such as AFLP, PCR, cloning, genome walking etc. which are all available to the skilled person. After cloning of an R-gene, said R-gene needs to be transferred to an at least partly Phytophthora susceptible plant.

There are multiple ways in which a recombinant nucleic acid (encoding an R-gene) can be transferred to a plant cell, for example Agrobacterium mediated transformation. However, besides by Agrobacterium infection, there are other means to effectively deliver DNA to recipient plant cells when one wishes to practice the invention. Suitable methods for delivering DNA to plant cells are believed to include virtually any method by which DNA can be introduced into a cell, such as by direct delivery of DNA such as by PEG-mediated transformation of protoplasts (Omirulleh et al., 1993), by desiccation/inhibition-mediated DNA uptake (Potrykus et al., Mol. Gen. Genet., 199:183-188, 1985), by electroporation (U.S. Pat. No. 5,384,253), by agitation with silicon carbide fibers (Kaeppler et al., 1990; U.S. Pat. No. 5,302,523; and U.S. Pat. No. 5,464,765), and by acceleration of DNA coated particles (U.S. Pat. No. 5,550,318; U.S. Pat. No. 5,538,877; and U.S. Pat. No. 5,538,880). Through the application of techniques such as these, certain cells from virtually any plant species may be stably transformed, and these cells developed into transgenic plants.

In case Agrobacterium mediated transfer is used, it is preferred to use a substantially virulent Agrobacterium such as A. tumefaciens, as exemplified by strain A281 or a strain derived thereof or another virulent strain available in the art. These Agrobacterium strains carry a DNA region originating from the virulence region of the Ti plasmid pTiBo542 containing the virB, virC and virG gees. The virulence (vir) gene products of A. tumefaciens coordinate the processing of the T-DNA and its transfer into plant cells. Vir gene expression is controlled by virA and virG, whereby virA upon perception of an inducing signal activates virG by phosphorylation. VirG, in turn, induces the expression of virB,C,D,E. These genes code for proteins involved in the transfer of DNA. The enhanced virulence of pTiBo542 is thought to be caused by a hypervirulent virG gene on this Ti plasmid (Chen et al. Mol. Gen. Genet 230: 302-309, 1991).

After transfer of a nucleic acid into a plant or plant cell, it must be determined which plants or plant cells have been provided with said nucleic acid. This is for example accomplished by using a selectable marker or a reporter gene. Among the selective markers or selection genes that are most widely used in plant transformation are the bacterial neomycin phosphotransferase genes (nptI, nptII and nptIII genes) conferring resistance to the selective agent kanamycin, suggested in EP131623 and the bacterial aphIV gene suggested in EP186425 conferring resistance to hygromycin. EP 275957 discloses the use of an acetyl transferase gene from *Streptomyces viridochromogenes* that confers resistance to the herbicide phosphinotricin. Plant genes conferring relative resistance to the herbicide glyphosate are suggested in EP218571. The resistance is based on the expression of a gene encoding 5-enolshikimate-3-phosphate synthase (EPSPS) that is relatively tolerant to N-phosphomethylglycine. Certain amino acids such as lysine, threonine, or the lysine derivative amino ethyl cysteine (AEC) and tryptophan analogs like 5-methyl tryptophan can also be used as selective agents due to their ability to inhibit cell growth when applied at high concentration. In this selection system expression of the selectable marker gene results in overproduction of amino acids by transgenic cells which permits the transgenic to grow under selection. Suitable examples of reporter genes beta-glucuronidase (GUS), beta-galactosidase, luciferase and green fluorescent protein (GFP).

WO 03/010319 describes a method for obtaining marker-free plants comprising a recombinant nucleic acid comprising a T-DNA construct allowing for transfer of said construct into the genome of a plant cell, said construct provided with a foreign nucleic acid that is free of nucleic acid encoding a selective marker. This method has proven to be very useful.

It is also possible to use the to be transferred R-gene as a selection marker. Hence, the invention further provides a method for determining whether a plant has been provided with an identified and cloned R-gene by exposing the resulting plant to at least one effector of Phytophthora and determining the presence or absence of a reaction to said at least one effector.

The invention further provides an isolated R-gene obtainable by a method for identifying a resistance gene (R-gene) in Solanum comprising providing a set of defined Phytophthora effectors,
exposing a part of a Solanum plant to said set of defined effectors,
identifying the presence or absence of a reaction of said plant to an effector from said set of defined effectors,
transferring at least part of the nucleic acid sequence of said Solanum to another plant
and testing the obtained plant or progeny thereof for an acquired reaction against at least one effector from said set of defined effectors, wherein said transferring is preceded by identifying said R-gene and by isolating said gene from said Solanum plant.

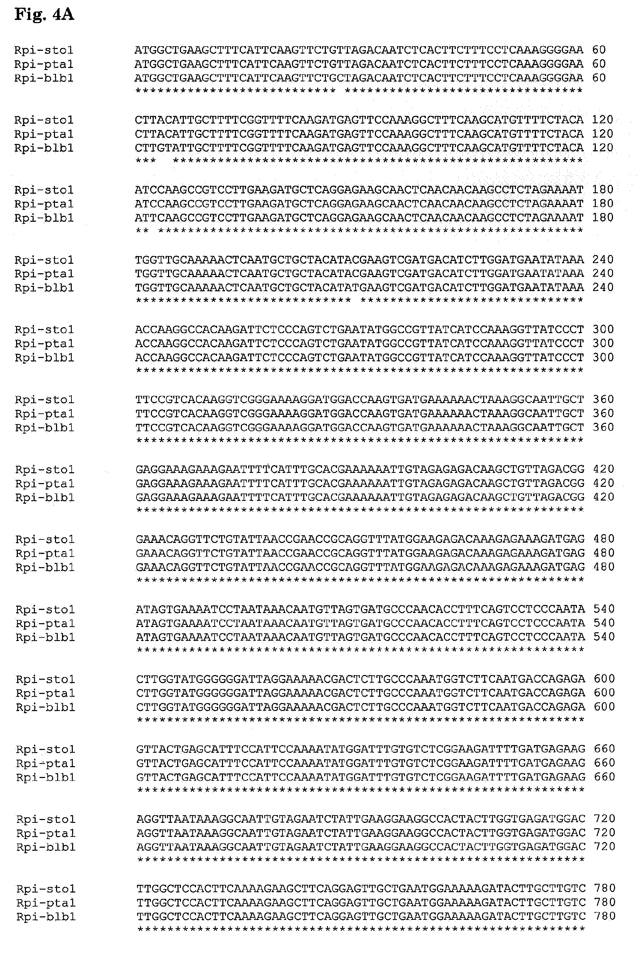

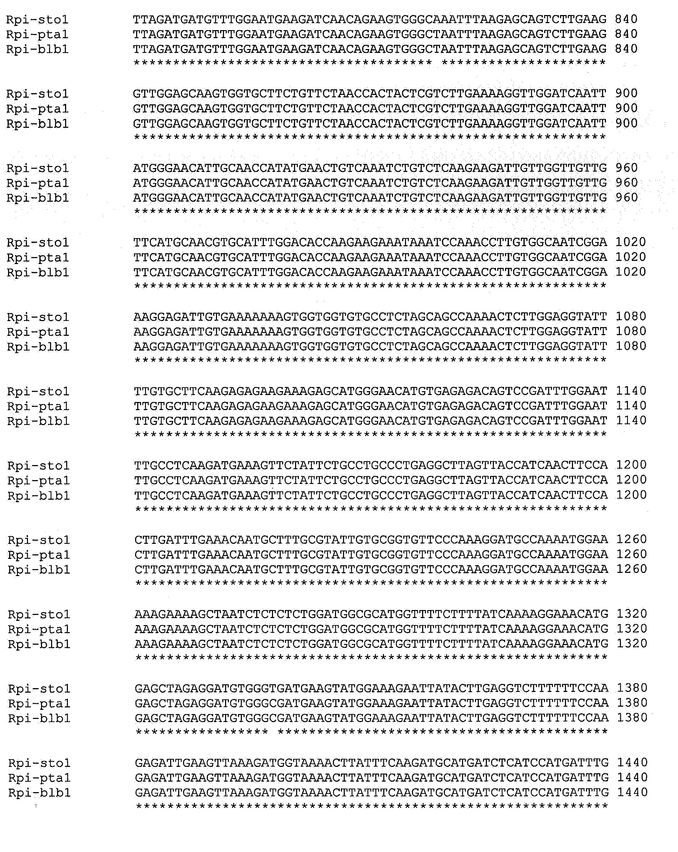

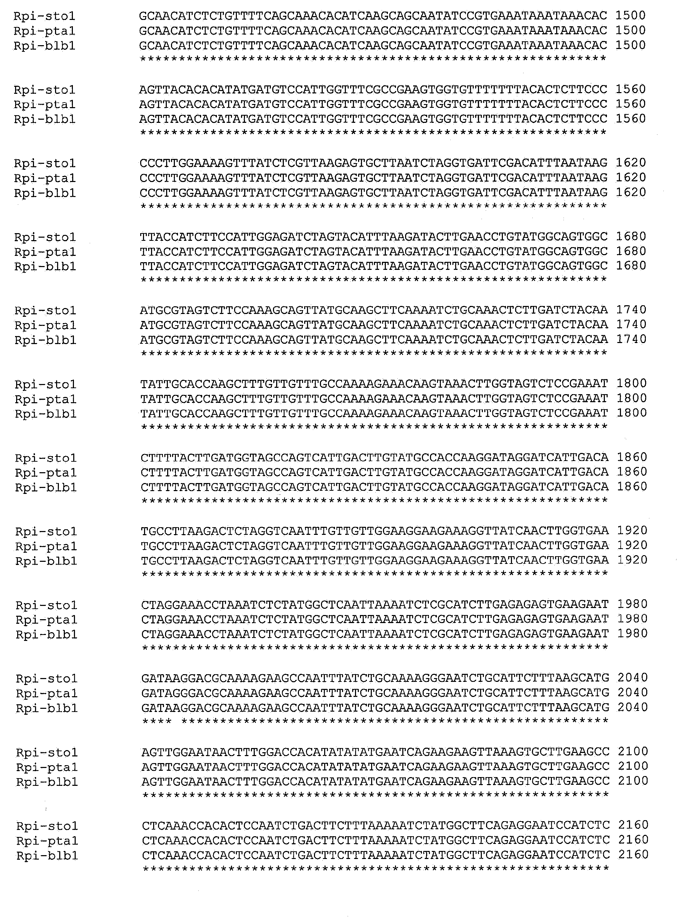

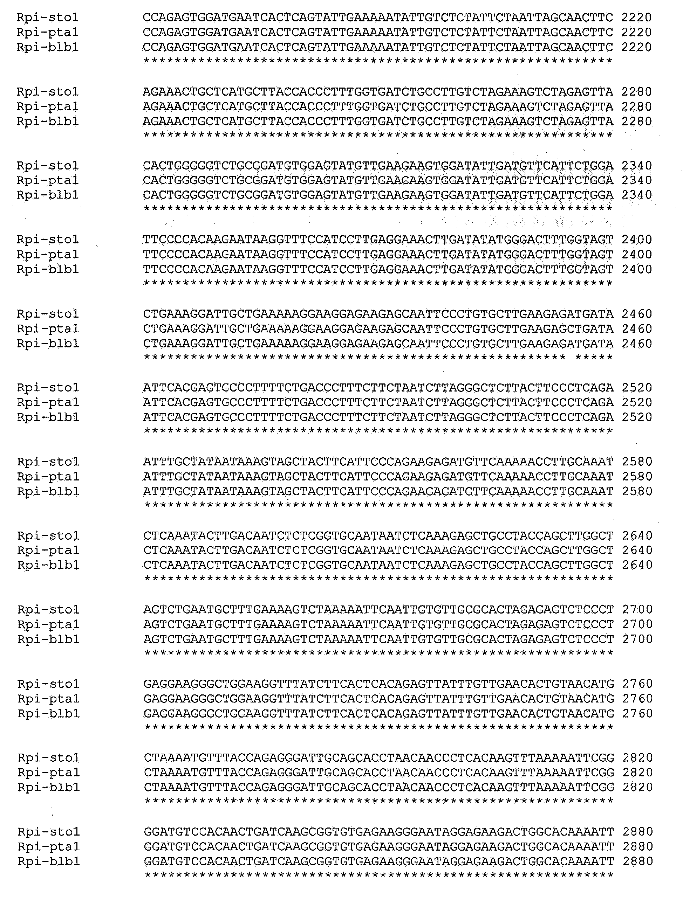

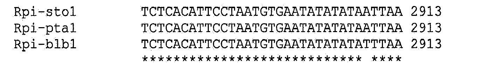

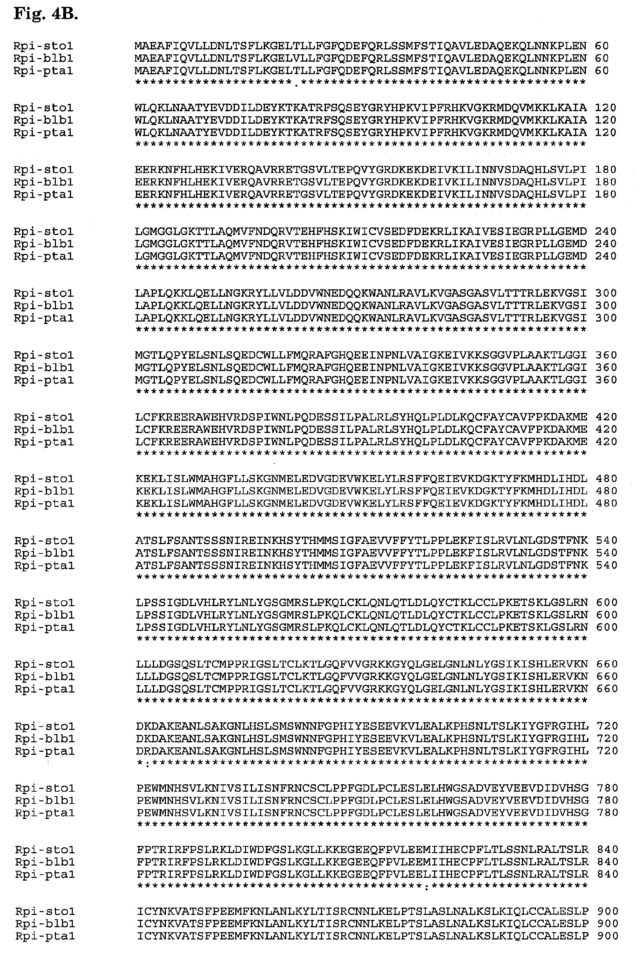

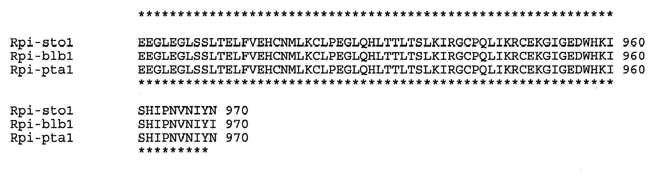

In a preferred embodiment, the invention provides an R-gene which is isolated from Solanum stoloniferum or Solanum papita and even more preferably said R-gene is Rpi-sto1 or Rpi-pta1. The sequences of these R-genes are shown in Figure 4.

The Rpi-sto1 and Rpi-pta1 genes/proteins are highly homologous to Rpi-blb1. The fact that 3 more or less the same genes are present in 3 different potato varieties is for example very useful in classical breeding. Now the classical breeder can select from 3 different varieties to introduce the same R-gene in another variety. The selection is for example based on the crossability of S. bulbocastanum, S. stoloniferum and S. papita with said another variety. Hence, the invention also provides a method for identifying alternative Solanum sources of a particular R-gene comprising the steps of any of the above described methods.

In yet another embodiment, the invention provides a method for determining whether an R-gene from Solanum provides resistance to a variety of Phytophthora isolates, comprising providing a plant with said R-gene and testing said plant for a reaction to a defined set of effectors, representing said variety of isolates. This method avoids the use of multiple Phytophthora isolates. Such a method provides a better resolution compared to use of one or multiple Phytophthora isolate(s). In a preferred embodiment, the invention provides a method for determining whether an R-gene from Solanum provides resistance to a variety of Phytophthora isolates, comprising providing a plant with said R-gene and testing said plant for a reaction to a defined set of effectors, representing said variety of isolates, wherein at least one said effectors is an elicitor.

Such a method is also very useful in respect of classical breeding in that it can be used to more easily select a suitable variety for crossings.

The invention further provides a method for determining whether a set of R-genes from Solanum provides resistance to a variety of Phytophthora pathotypes, comprising providing a plant with said set of R-genes and testing said plant for a reaction to a defined set of effectors, representing said variety of pathotypes. This method is very useful in testing the effect of stacked R-genes.

In another embodiment, the invention provides a method for determining whether a set of effectors is representative for a variety of Phytophthora isolates, comprising identifying a plant which gives a reaction against said set of effectors and testing whether said plant is resistant against said variety of isolates. If so, the set of effectors is representative and can be used instead of multiple Phytophthora isolates. By using a set of effectors in stead of a variety of Phytophthora isolates the reproducibility of any test using said set of effectors is greatly enhanced. Depending on for example environmental circumstances the effectors produced by a certain Phytophthora strain may vary. By using a set of elicitors lack of inconsistency can be prevented and experiments can be compared more reliable not only in a genetic modification approach but also in a classical breeding approach. In a preferred embodiment, the invention provides a method for determining whether a set of effectors is representative for a variety of Phytophthora isolates, comprising identifying a plant which gives a reaction against said set of effectors and testing whether said plant is resistant against said variety of isolates, wherein at least one of said effectors is an elicitor.

From an epidemiological point of view it is wise to combine not only several but also complementary sets of R-genes (i.e. stacking of R-genes). Introduction of multiple R-genes into a single variety through classical introgression breeding is technically possible but time consuming. A much faster and more flexible approach is the introduction through genetic modification. The invention also provides a method for providing a potato plant with a combination of resistance genes which provides resistance against a variety of pathotypes of Phytophthora, comprising providing said plant with at least two R-genes that together provide resistance against said variety of pathotypes, said R-genes being identifiable/identified by a method as described above, i.e. a method for determining whether an R-gene from Solanum provides resistance to a variety of Phytophthora pathotypes.

Preferably, at least one gene of said combination of resistance genes is *Rpi-blb3* (or *Rpi-sto1* or *Rpi-pta1*) or a functional fragment or a functional homologue thereof. Figure 5 shows the nucleotide sequence of *Rpi-blb3.* Figure 4 discloses the *Rpi-stol* and *Rpi-pta1* sequences.

The term "functional fragment thereof'' is typically used to refer to a fragment of the Rpi-blb3 (or *Rpi-stol* or *Rpi-pta1)* protein that is capable of providing at least partial resistance or increasing resistance in a plant of the Solanaceae family against an oomycte infection. Such a fragment is a truncated version of the Rpi-blb3 (or Rpi-sto1 or Rpi-pta1) protein as presented in Figure 4 or 5. A truncated version/fragment of for example the Rpi-blb3 protein is a fragment that is smaller than 847 amino acids and preferably comprises the larger part of the LRR domain (i.e. the larger part of the leucine-rich repeats domain) and/or the N-terminal parts of the Rpi-blb3 protein.

The term "functional homologue" is typically used to refer to a variant of the herein described Rpi-blb3 protein, which variant is capable of providing at least partial resistance or increasing resistance in a plant of the Solanaceae family against an oomycte infection. Included are artificial changes or amino acid residue substitutions that at least partly maintain the effect of the Rpi-blb3 protein. For example, certain amino acid residues can conventionally be replaced by others of comparable nature, e.g. a basic residue by another basic residue, an acidic residue by an acidic residue, a hydrophobic residue by another hydrophobic residue, and so on. Examples of hydrophobic amino acids are valine, leucine and isoleucine. Phenylalanine, tyrosine and tryptophan are examples of amino acids with an aromatic side chain and cysteine as well as methonine are example of amino acids with sulphur-containing side chains. Serine and threonine contain aliphatic hydroxyl groups and are considered to be hydrophilic. Aspartic acid and glutamic acid are examples of amino acids with an acidic side chain. In short, the term "functional homologue thereof' includes variants of the Rpi-blb3 protein in which a portion of the amino acids have been replaced conventionally and which at least partly maintain the effect of the Rpi-blb3 protein (i.e. at least partly providing or increasing resistance in a plant of the Solanaceae family against an oomycte infection). Also included in the term "functional homologue thereof" are homologous sequences. Preferably, such a homologue has at least 40% identity on the amino acid level. More preferably, the amino acid homology percentage is at least 86 or 90%. Even more preferably are amino acid homology percentages of 91, 92, 93, 94 or 95%. Most preferred are amino acid homology percentages of 96, 97, 98 or 99%.

A homologous nucleic acid sequence is a nucleic acid sequence that encodes for a homologous protein as described above.

Homology percentages can for example be determined by using computer programs such as BLAST, GAP, ClustalX or Clustal W.

Many nucleic acid sequences code for a protein which is 100% identical to the protein as presented in Figure 5. This is because the third nucleotide in a nucleotide triplet may vary without changing the corresponding amino acid (wobble position in the nucleotide triplets). Thus, without having an effect on the amino acid sequence of a protein the nucleotide sequence coding for this protein can be varied. However, in a preferred embodiment, the invention provides an isolated or recombinant nucleic acid sequence as depicted in Figure 4 or 5.

Fragments as well as homologues of the herein described Rpi-blb3 gene and protein can for example be tested for their functionality by using Agrobacterium tumefaciens transient assays (ATTA) on tobacco or potato or by using detached leaf assay.

The experimental part for example describes a functional screen for testing candidate homologues using *Agrobacterium tumefaciens* transient assays (ATTA) whereby 4 week old wild type *Nicotiana benthamiana* plants are infiltrated either with equal mixtures of two *Agrobacterium* strains containing the candidate *Rpi-blb3* homologue and the silencing suppressor p19 (Voinnet et al., 2003), or only the candidate Rpi-blb3 candidate. The infiltrated leaves are subsequently challenged one day after infiltration with a *P*. *infestans* strain, for example PY23, IPO-0 or 90128, in detached leaf assays. This system is equally suitable for testing candidate homologues of Rpi-blb3.

Transient gene expression is a fast, flexible and reproducible approach to high-level expression of useful proteins. In plants, recombinant strains of Agrobacterium tumefaciens can be used for transient expression of genes that have been inserted into the T-DNA region of the bacterial Ti plasmid. A bacterial culture is vacuum-infiltrated into leaves, and upon T-DNA transfer, there is ectopic expression of the gene of interest in the plant cells. However, the utility of the system is limited because the ectopic protein expression ceases after 2-3 days. It is shown that post-transcriptional gene silencing (PTGS) is a major cause for this lack of efficiency. A system based on co-expression of a viral-encoded suppressor of gene silencing, the p19 protein of tomato bushy stunt virus (TBSV), prevents the onset of PTGS in the infiltrated tissues and allows high level of transient expression. Expression of a range of proteins was enhanced 50-fold or more in the presence of p19 so that protein purification could be achieved from as little as 100 mg of infiltrated leaf material. Although it is clear that the use of p19 has advantages, an ATTA without p19 can also be used to test the functionality of candidate fragments and homologues.

Alternatively, each candidate gene (being a fragment or homologue) construct is targeted for transformation to a susceptible potato cultivar for example Desiree. Primary transformants are challenged in detached leaf assays using for example isolates IPO-0 and 90128. Transformants that are resistant to these isolates harbour functional fragments or homologues of Rpi-blb3.

The invention also provides a method for providing a potato plant having acquired resistance to a variety of Phytophthora pathotypes through the introduction of an R-gene with additional resistance against at least one additional pathotype of Phytophthora, comprising providing said plant with at least one further R-gene obtainable/obtained by a method as described herein, i.e. a method for identifying a resistance gene (R-gene) in Solanum comprising providing a set of defined Phytophthora effectors,
exposing a part of a Solanum plant to said set of defined effectors,
identifying the presence or absence of a reaction of said plant to an effector from said set of defined effectors,
transferring at least part of the nucleic acid sequence of said Solanum to another plant
and testing the obtained plant or progeny thereof for an acquired reaction against at least one effector from said set of defined effectors.

Preferably, said further R-gene is *Rpi-blb3* (or *Rpi-stol* or *Rpi-pta1)* or a functional fragment or a functional homologue thereof.

The invention further provides a plant obtainable by the method according to the methods as described above, i.e:
(i) a method for providing a potato plant with a combination of resistance genes which provides resistance against a variety of pathotypes (or strains or isolates) of Phytophthora, comprising providing said plant with at least two R-genes that together provide resistance against said variety of pathotypes, said R-genes being identifiable/identified by a method as described above, i.e. a method for determining whether an R-gene from Solanum provides resistance to a variety of Phytophthora pathotypes or
(ii) a method for providing a potato plant having acquired resistance to a variety of Phytophthora pathotypes through the introduction of an R-gene with additional resistance against at least one additional pathotype of Phytophthora, comprising providing said plant with at least one further R-gene obtainable/obtained by a method as described herein, i.e. a method for identifying a resistance gene (R-gene) in Solanum comprising
providing a set of defined Phytophthora effectors,
exposing a part of a Solanum plant to said set of defined effectors,
identifying the presence or absence of a reaction of said plant to an effector from said set of defined effectors,
transferring at least part of the nucleic acid sequence of said Solanum to another plant
and testing the obtained plant or progeny thereof for an acquired reaction against at least one effector from said set of defined effector.

Preferably, said at least one gene of said combination of resistance genes is *Rpi-blb3* (or *Rpi-sto1* or *Rpi-pta1)* or said further R-gene is *Rpi-blb3* (or *Rpi-sto1* or *Rpi-pta1*)*.* Another preferred R-gene is R3a.

Preferably, the plant obtainable by the present invention comprises an R-gene which is foreign to the host cell. The term "foreign" is herein used to describe the situation in which the R-gene is heterologous with respect to the host cell (i.e. derived from a cell or organism with a different genomic background) or the R-gene is homologous with respect to the used host cell but located in a different genomic environment than the naturally occurring counterpart of R-gene.

Even more preferably, an introduced R-gene is a so-called cisgene i.e. a gene obtained/derived/isolated from a same species or a crossable species. Preferably, said cisgene is under control of native 5' (promoter) and 3' (terminator) sequences and even more preferably said cisgene is a genomic sequence. Said genomic sequence can contain intron and exon sequences.

Preferably, an obtained genetically modified Solanum plant comprises Solanum 5' and 3' sequences to direct transcription of a cDNA sequence or a genomic sequence. The used cDNA or genomic sequence may be orientated sense or antisense in relation to the used 5' and 3' sequences. The 5' and 3' sequences can be originally linked to the used cDNA or genomic sequence or they can normally be linked to another coding sequence in a Solanum. In a preferred embodiment, the used coding sequence is a genomic sequence (in sense or antisense orientation) and the used 5' and 3' sequences are the ones that are linked to the genomic sequence under normal conditions (i.e. the 5' and 3' sequences are in operable linkage with their natural coding sequence).

*Rpi-blbl* was long time considered to provide full Phytophthora infestans protection. However, the protection provided by Rpi-blb1 has been broken by at least one pathotype (isolate PIC99189). The methods of the present invention provide new R-genes or homologues of known R-genes. The R-genes obtainable by the methods of the invention can be introduced in (partially) susceptible Solanum or Lycopersicon (potato or tomato). Preferably, multiple (for example two or three), different R-genes are introduced. The effect of such multiple R-genes constructs can easily be tested by using the complementary or differentially effectors instead of using Phytophthora isolates. Moreover, the skilled person has now been provided with all the tools necessary to check whether "new" Phytophthora isolates will result in a Phytophthora epidemic on for example certain Solanum fields or whether said field are resistant. This is for example tested by cloning the cDNAs of the effectors that interact with introduced R genes, of said "new" Phytophthora strain and testing whether they give a reaction on a certain Solanum that has been genetically modified with one or multiple R-genes.

In yet another embodiment, the invention provides a method for selecting a plant that is to be used in breeding, comprising exposing a part of a plant to at least one elicitor and determining the presence or absence of a reaction to said at least one elicitor.

Such a method is very useful in the field of classical breeding and provides a means to select the starting material in a crossing (i.e. the parents). Such a method of the invention is preferred over the use of a method that uses identification of markers, because the present method focuses on functionality instead of presence. Such a method also allows selection of plants with identical reaction (i.e. resistance) to an elicitor but which plants have different properties in respect of crossability.

The invention will be explained in more detail in the following, nonlimiting examples.

### Experimental part

### Materials and Methods

### Plant material

All plant material is maintained *in vitro* on Murashige and Skoog (MS) medium supplemented with 30 g per liter sucrose (MS30) at 24°C and 16 h light/8 h dark. Shoots or single node cuttings are transferred to fresh MS30 medium every 3 to 4 months for maintenance.

### Phytophthora infestans isolates

A broad range of *P. infestans* isolates is used. Isolates were collected from various sources, and were assessed for their aggressiveness on potato leaves, zoospore formation in the lab, etc). Also the isolates are inoculated on the differential set, to determine the avirulence profile.

A set of 11 aggressive isolates was selected, which originate from different geographic locations and vary in their mating type and avirulence profile (Table 2).

### Disease assays

### A. In vitro assay

The development of the in vitro assay for disease testing is described in (Huang *et al.,* 2005), and will be summarized below.

To prepare plantlets for *in vitro* inoculation, fresh shoots or single nodes were cut and transplanted to glass jars (diameter 10 cm) or plastic jars (diameter 15 cm) with MS30. Each jar contained 5 cuttings. A 2 to 3 cm space was left between the cuttings and the inner wall of the jars. *In vitro* plantlets were grown at 24°C and 16 h light/8 h dark. Plantlets with 3 to 4 fully developed leaves were used for *in vitro* inoculation. Depending on the potato genotype, it took 2 to 4 weeks from transplanting to inoculation. Generally, it took about 4 to 5 weeks for in vitro sown seedlings to develop enough leaf material for inoculation.

The 3 largest leaves of each *in vitro* plantlet were inoculated (1 spot per leaf) by pipetting 10 µl droplets of a zoospore suspension of 2.5 x 10⁴ spores/ml on the adaxial side. Inoculum preparation and inoculation were performed in sterilized conditions. Leaves touching the inner wall or the lid of the jars or the medium were excluded because we found these leaves to be more susceptible than others of the same plants. The jars with inoculated in vitro plantlets were incubated in the same climate chamber as the trays with inoculated detached leaves.

Susceptible interactions were scored as S (spreading lesion with massive sporulation) or class SQ (spreading lesion with no or little sporulation). Resistant interactions were scored as class R (no symptom or localized HR-like necrosis) or class RQ (trailing HR necrosis). Rarely, intermediate phenotypes (class Q) between compatible and incompatible ones were observed, such as sporulation on 2 to 3 plantlets per jar and localized HR on the other plantlets.

### B. Detached leaf assay

The detached leaf assay has been described in (Vleeshouwers et al., European Journal of Plant Pathology 105: 241-250, 1999), and is summarized below.

For propagation, shoots or single nodes were cut and transferred to fresh MS30 medium. After one week of rooting, the in vitro plantlets were transferred into pots of sterilized soil and placed in a climate chamber to allow a progressive adaptation to lower humidity. The plants were then grown in the greenhouse. Leaves from plants of 6 to 8 weeks old were cut for inoculation.

The 3rd to 5th fully developed leaves (counted from the top) were cut from greenhouse-grown plants and placed in water saturated florists foam (Oasis®, Grünstadt, Germany) in a tray. Depending on the shape and size of the *Solanum* leaves, one or more leaves were used for inoculation. In general, 10 spots were inoculated per genotype by pipetting 10 µl droplets of a zoospore suspension of 5 x 10⁴ spores/ml on the abaxial side. The trays were then covered with transparent lids (covered trays), transferred into a climate chamber, and incubated at a 16h/8h day/night photoperiod at 16°C.

Resistance level was determined by determining the lesion size (LS), macroscopic scoring, or a combination of both. LS were measured usually at day 6 after spot inoculation using an electronic caliper connected to a computer. The mean LS was calculated from 10 replicates. Based on the average lesion size, a relative score from 0-9 was assigned, relative to Bintje. Also the lesion phenotype was examined. In addition macroscopic scoring was carried out as follows.

Compatible interactions were classified as S (susceptible, spreading lesion with massive sporulation) or SQ (spreading lesion with no or little sporulation). Incompatible interactions were scored as R (Resistant, no symptom or localized HR-like necrosis) or RQ (trailing HR necrosis). Also intermediate phenotypes (Q) between compatible and incompatible ones were observed, such as sporulation on 2 to 3 leaflets per compound leaf and localized HR on the other leaflets.

### Agroinfection

Recombinant A. tumefaciens GV3101 strains carrying candidate effectors (obtained from OSU) in pGR106 were used to screen for a response in Solanum. The pGR106-CRN2 and the pGR106 empty vector (Torto et al., Genome Res. 13: 1675-1685, 2003) were used as a positive and negative control respectively. Cultures were grown for 2 days at 28°C on solid agar LB medium supplemented with antibiotics. Excess of bacteria was inoculated by piercing the leaf at both sides of the mid-vein. Local and systemic symptoms were visually scored every 2-4 days. For mature plant inoculations, usually three leaves from three to four week old plants was used, and the leaf age was rotated for replicates of the various treatments.

### Application of agroinfection in Solanum

Resistance to PVX is known to occur in *Solanum* and would interfere with large-scale screenings with the binary PVX vector. To determine the extent to which the assay is applicable to the diverse *Solanum* germplasm, we inoculated plants corresponding to 80 *Solanum* clones from 31 species with *A. tumefaciens* carrying pGR106 and pGR106-CRN2 (Vleeshouwers et al., Molecular Plant Pathology 7, 499-510, 2006). Treatment with the pGR106-CRN2 strain consistently caused necrosis around the inoculation sites on 50 plant clones (63%) whereas the empty vector strain caused no symptoms. We concluded that these plants are suitable for PVX agroinfection assays. The remainder of the *Solanum* clones tested were not suitable for the assay. Responses to both the positive and negative controls occurred in 20 clones and were regarded as nonspecific reactions to PVX. Ten other clones showed no necrotic response to pGR106-CRN2 possibly because the gene insert was not expressed *in planta.* In summary, it appears that the PVX assay is suitable for about 80% of the species examined and 60% of the clones. Therefore, before each screening with a large set of candidate effectors from *P. infestans,* a pre-screening with pGR106-CRN2 and the pGR106 empty vector is performed.

### Agroinfiltration (ATTA)

Coinfiltrations of Agrobacterium strains carrying the R gene and the AVR gene were performed in *N. benthamiana.* Recombinant A. tumefaciens cultures were grown in LB medium (10 gram bacteriological peptone, 10 gram NaCl and 5 gram yeast extract in 1 liter MQ water) supplemented with 50 mg/L Rifampicilin and 50 mg/L Kanamycin for the LBA4404 constructs. The COR308 was supplemented with 5 mg/ L tetracycline and 50 mg/L kanamycin (the empty COR308 was only grown on tetracycline). After one or two days a calculated amount of culture (according to OD 0.5 at 600 nm) was transferred to YEB medium (5 gram beef extract, 5 gram bacteriological peptone, 5 gram sucrose, 1 gram yeast extract, 2ml 1 M MgSO4 in 1 liter MQ water) supplemented with Kanamycin for all strains, but for COR308 empty vector tetracycline was used. After 1 day overnight cells were centrifuged at 3500 rpm and resuspended in MMA medium (20 gram sucrose, 5 gram MS salts and 1.95 gram MES) supplemented with 1 ml 200 mM acetosyringone to a final OD of 0.5. Both constructs (R-gene and PEX) were mixed 1:1 before infiltration into 4-6 weeks old plants with a 3ml syringe. Symptom development was monitored after 5 to 6 days.

### Examples

### Screening of resistant Solanum spp for response to RXLR effectors

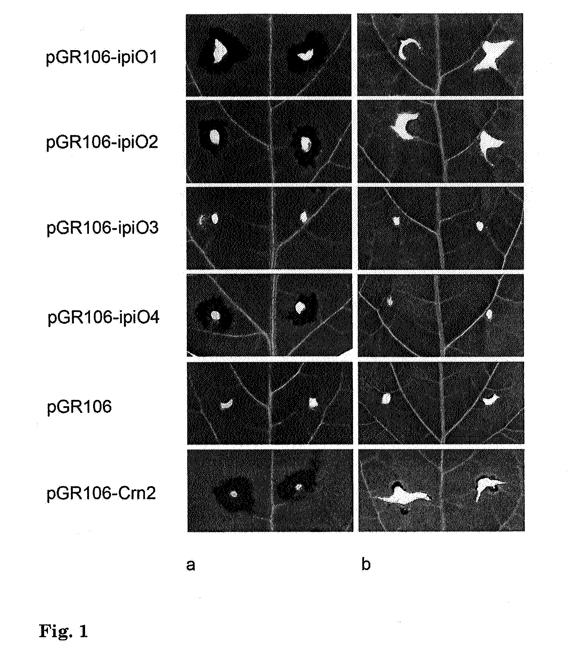

Wild tuber-bearing *Solanum* species bearing representatives of all taxonomic series from *Solanum* sect Petota were assessed for resistance to *P*. *infestans* isolate 90128 using a high-throughput disease assay *in vitro* and on detached leaves. Identified resistant genotypes were subjected to functional screenings with a transient expression system based on *Agrobacterium tumefaciens* and potato virus X (PVX) expressing candidate effector genes of *P. infestans.* Various potential R-AVR interactions were identified. Some effector candidates evoked a response in a broad variation of *Solanum* genotypes. Other effector candidates were very specific and only elicited necrosis in one definite genotype, e.g. RD7 (Avr3a family) in Desiree-R3a. Also RD6, representing the ipiO family, revealed a strong and specific response in Sto17605-4 and this combination was chosen for further analysis as potential AVR-R combination (Figure 1).

### Identification of the resistance gene Rpi-Stol in S. stoloniferum

Genetic dissection of the resistance was performed by crossing the resistant autotetraploid Sto17605-4 with the susceptible diploid Tbr RH89-039-16 (RH), and testing the population for resistance to *P. infestans* isolate 90128. Six days after inoculation, fourteen progeny genotypes showed large, extensively sporulating lesions whereas the nineteen other genotypes displayed small localized HR spots at the inoculation sites (Table 3A). The 1:1 segregation suggests a monogenic inheritance of a dominant R gene, hereby designated *Rpi-sto1.*

### Response to ipiO effectors cosegregates with Rpi-Stol resistance

To examine whether *Rpi-Stol* co-segregates with the response to IPIO, both parents and offspring genotypes from the Sto17605-4 x RH population were infected with Agrobacterium strains carrying pGR106-IPIOK143N and -IPIO2 (Table 3B). All late blight resistant plants showed local necrosis to pGR106-IPO PVX agroinfection, and all susceptible plants remained devoid of symptoms. This co-segregation of response to pGR106-IPIO1K143N and - IPIO2 with resistance suggests that these IPIO alleles function as avirulence factors to RPI-STO1.

### Specificity of Rpi-Sto1 resistance

The resistance spectrum of *Rpi-stol* was examined by inoculating 12 *P. infestans* isolates on detached leaves (Table 4). Rpi-sto1 was effective to 11 isolates, yet one virulent isolate PIC99189 was identified. This isolate was also able to infect the entire population Sto17605-4 x RH, whereas the avirulent isolates IPO-C and USA618 followed the same segregation pattern as isolate 90128 (Table 3A). These data suggest that 90128, IPO-C and USA618 express certain specific avirulence genes, which are not expressed in PIC99189.

### Specificity of ipiO avirulence genes

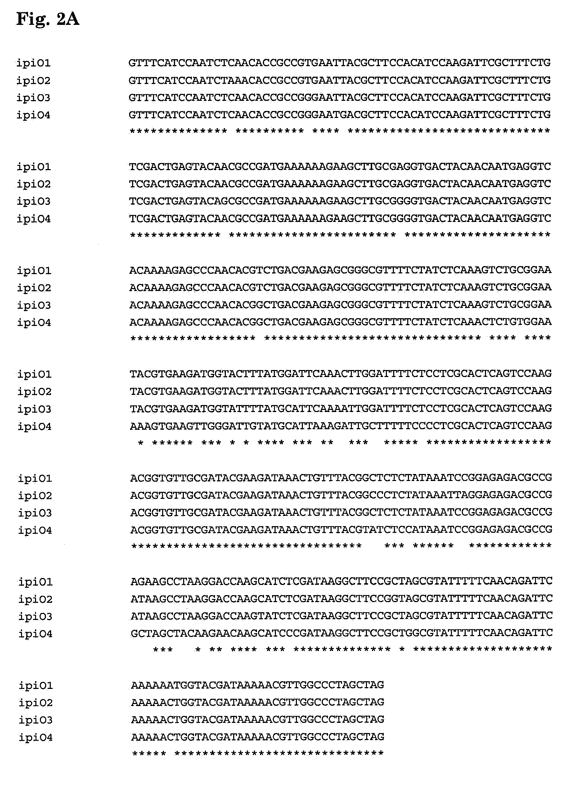

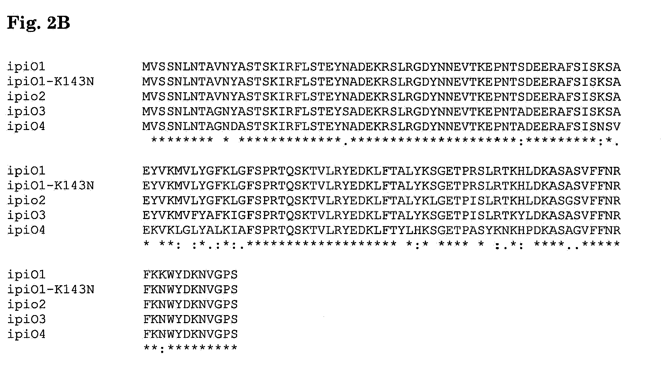

The *ipiO* alleles of the *P. infestans* isolates 90128, IPO-C, USA618 and PIC99189 were amplified using conserved primers IPIO-F and IPIO-R. Sequencing revealed that these isolates share 4 different ipiO alleles *ipiO1, ipiO2, ipiO3* and *ipiO4* (Figure 2A). The predicted proteins IPIO1 and IPO2 are highly similar, with only 4 amino acids different, whereas IPIO3 and IPIO4 contain 10 and 22 polymorphic residues respectively compared to ipiO1 (Figure 2B). The avirulent isolates on Rpi-Sto1 all contained *ipiO1, ipiO2,* or both, whereas the virulent isolate contained *ipiO3* and *ipiO4* (Table 3A).

All available IPIO were tested for response on the Sto17605-4 x RH population by agro-infection. As pGR106-IPIO1K143N and-IPIO2, also pGR106-IPIO co-segregated with the resistance, whereas no response to pGR106-IPIO3 and -IPIO4 could be detected in the population (Table 3B). In summary, these data indicate that *Rpi-sto1* and *ipiO* follow the gene-for-gene model with different specificities between various *ipiO* alleles.

### Comparative genomic characterization of Rpi-stol

To accelerate characterization of *Rpi-Sto1,* we applied sequence information of known R genes: an allele-mining study with *Rpi-blb1*/*Rb* was performed on a varying collection of *Solanums. Rpi*-*blb1*-specific primers AR0517 and ARO519 specifically amplified DNA fragments of the expected size in various wild *Solanum* species a.o. in the original Blb17692 accession, but also in Sto17605-4 and in Pta17831-8. Testing these same primers on the Sto17605-4 x RH population revealed that amplified fragment co-segregated with the resistance. The same was found for CT88, a genetic marker 0.3 cM linked to *Rpi-blbl* (Table 3C). Based on these genetic data, we hypothesized that *Rpi-stol* may be a homolog of *Rpi-blb1.*

### Rpi-stol is functionally homologous to Rpi-blbl

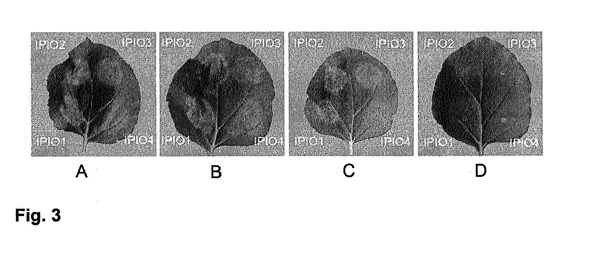

To test whether IPIO functionally interacts with Rpi-blb1, the hypothesized R-AVR interaction was reconstructed in a transient expression system in *Nicotiana benthamiana,* by co-infiltration of equal mixtures of A. *tumefaciens* strains carrying COR308-Rpi-blb 1 with A. *tumefaciens* strains carrying pGR106-IPIO1, -IPIO2, -IPIO3 or -IPIO4 in leaves. After 4-5 days, a confluent necrosis occurred in leaf panels in which pGR106-IPIO1 and -IPIO2 were co-infiltrated, whereas leaf panels with co-infiltrated pGR106-IPIO3 and -IPIO4 only showed a weak chlorosis or remained devoid of symptoms or (Figure 3A). Co-infiltration of A. *tumefaciens* strains carrying pGR106-IPIO1, -IPIO2, - IPIO3 and -IPIO4 with A. *tumefaciens* strains carrying COR308-Rpi-blb2 showed no response (Figure 3D). The observed symptoms in the agroinfiltration system provided functional evidence for interaction of Rpi-blb 1 with IPIO1 and IPIO2 and suggest that *Rpi-blbl* and *Rpi-stol* may represent homologous R genes.

### Cloning of Rpi-sto1 and Rpi-pta1

To clone homologues of *Rpi-blbl,* PCR products from Sto17605-4 and Pta17831-8 were amplified with primers GWPBlb1F (5'-CACCATGTTGTAATTATTGGCGAAC) and TBlb1R (5'-GTTGTTATAAGGGTATAAGTGAGC) and cloned into the pENTR directional TOPO vector. By subsequent restriction analysis of 96 individual clones from each genotype 5 different classes of *Rpi-blb1* homologues were identified from Sto17605-4 and Pta17831-8, respectively. Each unique class of candidate R genes was sequenced. Class A from both Sto and Pta appeared highly homologous to Rpi-blb1, with 99% sequence similarity at DNA and amino acid level (Figure 4).

For immediate functional analysis, clones from each class were recombined into the binary Gateway expression vector pK7WG2 and transferred to *A. tumefaciens.* Equal mixtures of two *A. tumefaciens* strains containing both the candidate *Rpi-blb1* homologue and one of the pGR106-IPIO clones were infiltrated in *N. benthamiana* leaves. Leaf panels containing co-infiltrated *A. tumefaciens* clones expressing class A homologues and pGR106-IPIO1 or -IPIO2 showed a clear confluent necrosis after 3-4 days (Figure 2B, C). This provided evidence that we cloned two functional *Rpi-blb1-*like genes from Sto and Pta, hereby designated *Rpi-stol* and *-pta1. Agrobacterium* clones containing the other candidate *Rpi-blb1-*like homologues did not induce any necrosis when co-infiltrated with *A. tumefaciens* clones containing any of the four pGR106-IPIO constructs.

### Tables

**Table 1. R-genes and quantitative trait loci for late blight resistance reported for wild Solanum species**

| **Wild species** | **Locus type or name** | **Chromosome** |
|---|---|---|
| S. berthaultii | QTLs (4) | I, III, VII and XI |
| | *Rpi-ber1* | X |
| | *Rpi-ber2* | VII |
| S. bulbocastanum | *RB*/*Rpi-blb1* | VIII |
| | *Rpi-blb2* | VI |
| | *Rpi-blb3* | IV |
| S. caripense | QTL (2) | unassigned |
| S. demissum | *R1* | V |
| | *R2* | IV |
| | *R3, R6, R7* | XI |
| | *R3a* | XI |
| | *R3b* | XI |
| | *R5-R11* | XI |
| | *R10, R11* | XI |
| S. microdontum | QTLs (3) | IV, V and X |
| | QTL | Unassigned |
| S. mochiquense | *Rpi-mcq1 (Rpi-moc1)* | IX |
| S. paucissectum | QTLs (3) | X, XI and XII |
| S. phureja | *Rpi-phul* | IX |
| S. pinnatisectum | *Rpi-pnt1 (Rpi1)* | VII |
| S. vernei | QTLs (several) | VI, VIII, IX |
| Hybrids with S. tuberosum | *Rpi-abpt* | IV |
| | *R2-like* | IV |
| | QTLs (several) | several |
| | QTLs | IV |

**Table 2 Characteristics of P. infestans isolates used in this application, including putative virulence profiles**

| **Isolate** | **Year** | **Geographic origin** | **Mating type** | **Obtained from** | **Virulence profile** |
|---|---|---|---|---|---|
| 90128 | 1990 | Geldrop, Netherlands | A1 | Govers, Phytopathology WUR | 1.3.4.7.(8) |
| USA618 | unkno wn | Toluca Valley, Mexico | A2 | Bill Fry, Cornell, USA | 1.2.3.6.7.11 |
| 89148-09 | 1989 | Netherlands | | Govers, Phytopathology WUR | 0 |
| IPO-C | 1982 | Gembloux, Belgium | A2 | Kessel, PRI, WUR | 1.2.3.4.5.6.7.(10.11) |
| VK98014 | 1998 | Veenkolonién, Netherlands | A1 | Kessel, PRI, WUR | 1.2.3.4.7.11 |
| NL00228 | 2000 | Dinteloord, Netherlands | A2 | Kessel, PRI, WUR | 1.2.3.4.7.(11) |
| EC1 | | Ecuador | | Birch, SCRI, Scotland | 1.3.4.7.(11) |
| F95573 | 1995 | Flevoland, Netherlands | A1 | Govers, Phytopathology, WUR | 1.3.4.7.10:11 |
| IPO-428-2 | 1992 | Ede, Netherlands | A2 | Kessel, PRI, WUR | 1.3.4.(6).7.8.11 |
| NL01096 | 2001 | Katshaar, Netherlands | A2 | Kessel, PRI, WUR | 1.3.4.7.(8).10.11 |
| PIC 99177 | 1999 | Metepec, Mexico | | Kessel, PRI, WUR (Flier *et al.,* 2002) | 2.(3.4).7.(11) |
| PIC 99183 | 1999 | Metepec, Mexico | | Kessel, PRI, WUR (Flier *et al.,* 2002) | 1.3.(4).7.(8.11) |
| PIC 99189 | 1999 | Metepec, Mexico | | Kessel, PRI, WUR (Flier *et al.,* 2002) | 2.(5.7.10).11 |
| PIC 99190 | 1999 | Metepec, Mexico | | Kessel, PRI, WUR (Flier *et al.,* 2002) | 1.3.4.7 |

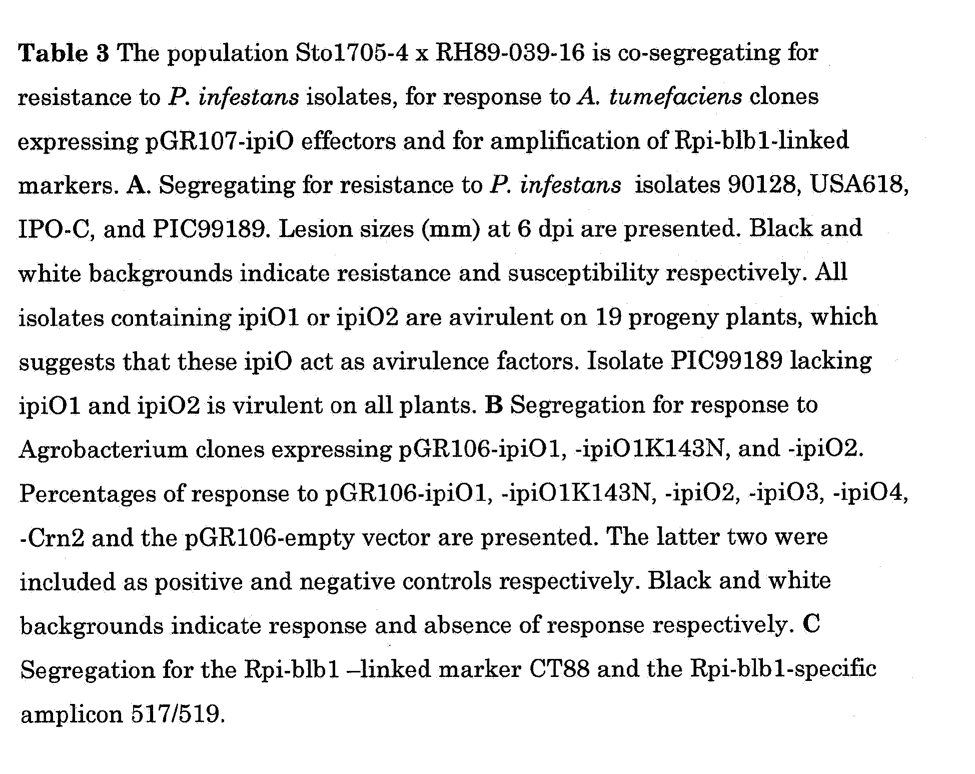

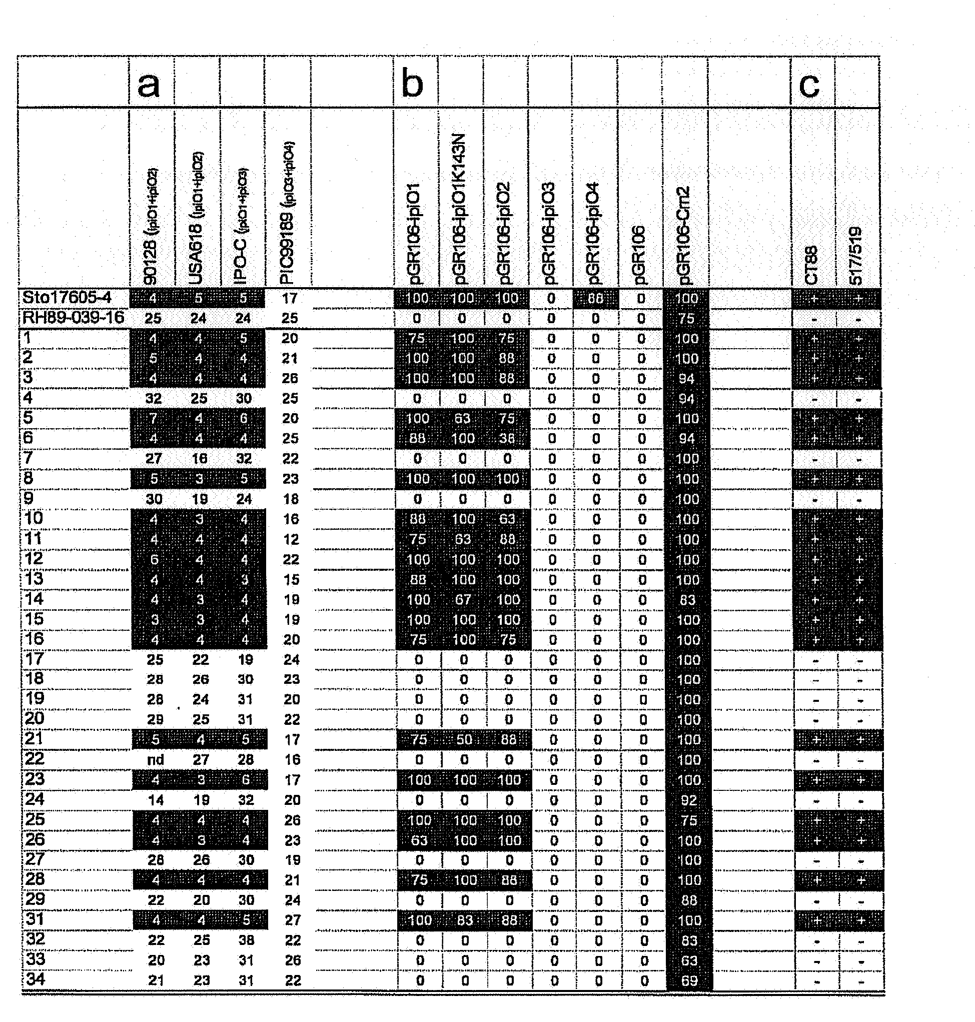

**Table 4. Resistance spectrum Sto17605-4 to P. infestans isolates. The geograpic origin, collection year, host plant and provider of the isolates are presented. Solanum stoloniferum (Sto) 17605-4 is resistant (R) to most tested isolates, except PIC99189. S. tubersoum (Tbr) RH89-039-16 is susceptible to all tested isolates.**

| Isolate | Origin | | | | Resistance | |
|---|---|---|---|---|---|---|
| | Collected in | Host | Year | Obtained from | Sto 17605-4 | RH89-039-16 |
| 90128 | Netherlands | Tbr | 1990 | Govers, WUR, Netherlands | R | S |
| USA618 | Mexico | Tbr | unknown | Bill Fry, Cornell, USA | R | S |
| 89148-09 | Netherlands | Tbr | 1989 | Govers, WUR, Netherlands | R | S |
| IPO-C | Belgium | Tbr | 1982 | Kessel, WUR, Netherlands | R | S |
| VK98014 | Netherlands | Tbr | 1998 | Kessel, WUR, Netherlands | R | S |
| NL00228 | Netherlands | Tbr | 2000 | Kessel, WUR, Netherlands | R | S |
| EC1 | Ecuador | | | Birch, SCRI, Scotland | R | S |
| F95573 | Netherlands | Tbr | 1995 | Govers, WUR, Netherlands | R | S |
| IPO-428-2 | Netherlands | Tbr | 1992 | Kessel, WUR, Netherlands | R | S |
| NL01096 | Netherlands | Tbr | 2001 | Kessel, WUR, Netherlands | R | S |
| PIC 99189¹ | Mexico | Sto | 1999 | Kessel, WUR, Netherlands | S | S |

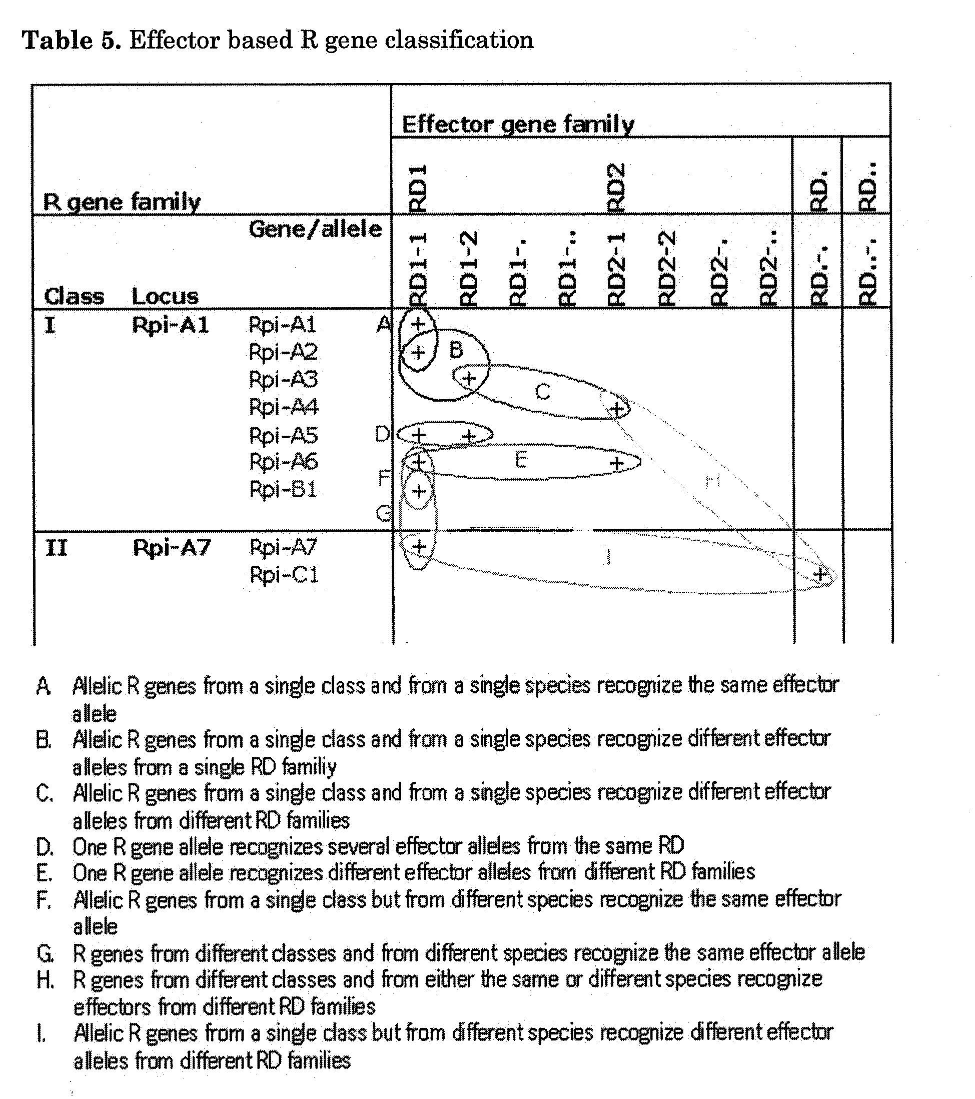

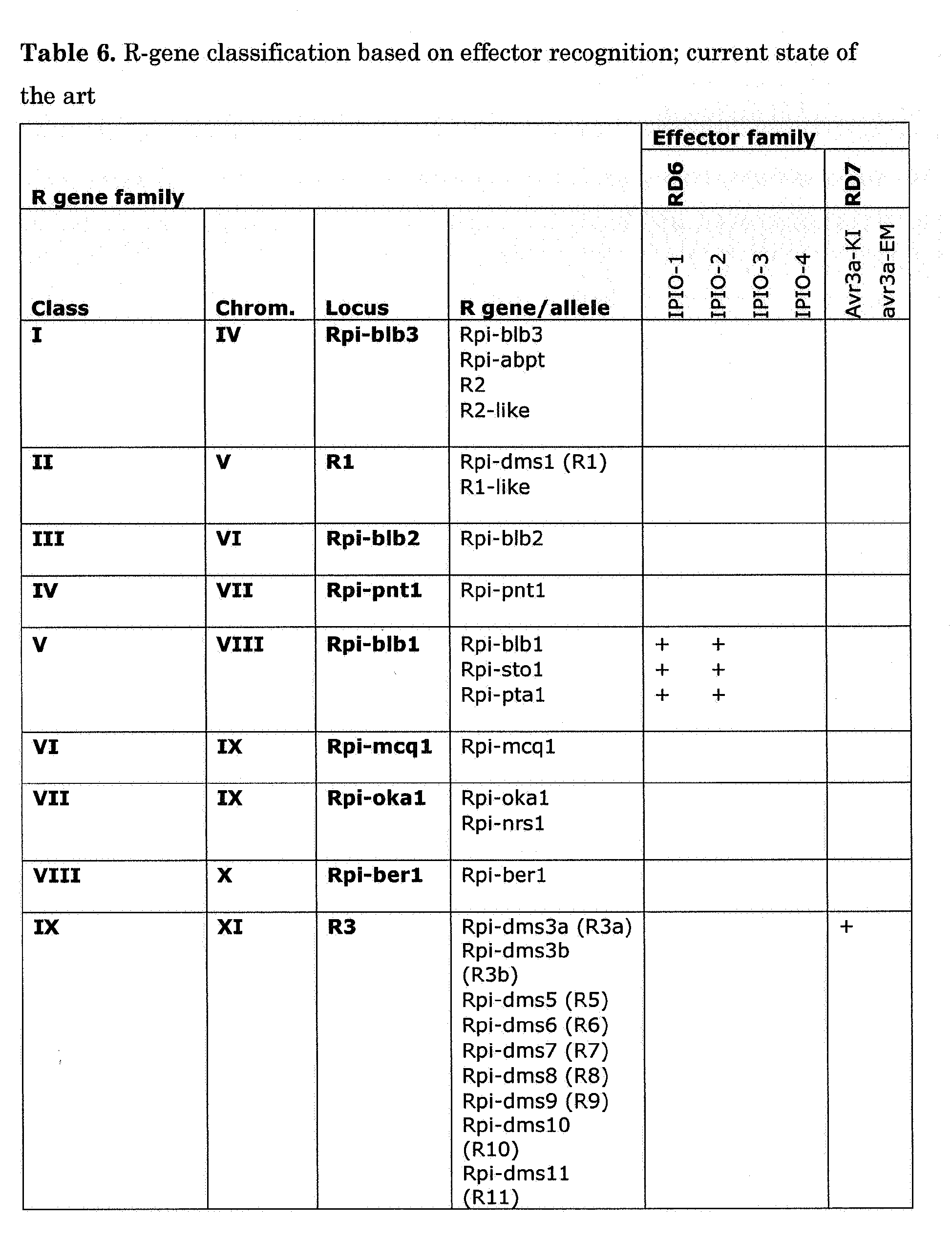

### Description of Figures

**Figure 1****.** PVX agroinfection shows specific responses to various pGR106-IPIO in *S*. *stoloniferum* 17605-4 **(a)** and not in *S. tuberosum* RH89-039-16 **(b).** At both sides of the vein, leaves were wound-inoculated with *A. tumefaciens* strains carrying pGR106, pGR106-IPIO1, -IPIO2, -IPIO3, -IPIO4, in addition to pGR106-CRN2 and the pGR106-empty vector as a positive and negative control respectively.
**Figure 2**. IPIO sequence alignments. A. DNA sequence alignment. B. Amino acid sequence alignment.
**Figure 3**. Functional reconstruction of the R-AVR interaction in N. benthamiana. (**a**) A. tumefaciens expressing pGR106-IPO1, -ipiO2, -ipiO3, or-IPIO4 were mixed in a 1:1 ration with A. tumefaciens strains COR308-Rpi-blb1 (a), COR308-Rpi-sto1 (b), COR308-Rpi-pta1 (c) or COR308-Rpi-blb2 (d) and infiltrated in N. benthamiana leaves. Pictures were taken 6 days after infiltration.
**Figure 4****.** Sequence alignment of Rpi-sto1 Rpi-pta1 and Rpi-blb1. A. DNA sequence alignment. B. Amino acid sequence alignment
**Figure 5****.** Sequence of Rpi-blb3. **A.** Nucleotide sequence of clone Blb25-B2 (8461 bp) containing the *Rpi-blb3* gene and regulatory sequences. The Rpi-blb3 coding region of 2544 bp is highlighted in lower case (2944-5487). The upstream 2732 nt (211-2942) and the downstream 882 nt (5488-6370) harbour the regulatory sequences **B.** Deduced Rpi-blb3 protein sequence. The amino-acid sequence deduced from the DNA sequence of Rpi-blb3 is divided into three domains (CC, NB-ARC and LRR). Hydrophobic residues in the CC domain are underlined. Conserved motifs in R proteins are written in italic in the NBS domain. Residues matching the consensus of the cytoplasmic LRR are indicated in bold in the LRR domain

## Claims

1. A method for identifying a resistance gene (R-gene) in Solanum comprising providing a set of defined Phytophthora effectors,
exposing a part of a Solanum plant to said set of defined effectors, identifying the presence or absence of a reaction of said plant to an effector from said set of defined effectors,
transferring at least part of the nucleic acid sequence of said Solanum to another plant
and testing the obtained plant or progeny thereof for an acquired reaction against at least one effector from said set of defined effectors.

2. A method according to claim 1, wherein said effector is an elicitor.

3. A method according to claim 1 or 2, wherein said transferring comprises crossing said Solanum plant with another Solanum plant.

4. A method according to claim 1 or 2, wherein said transferring is preceded by identifying said R-gene and by isolating said gene from said Solanum plant.

5. A method according to claim 4, wherein the obtained gene is transferred to another plant.

6. A method according to any one of claims 1 to 5, wherein said acquired reaction is an acquired resistance for a Phytophthora isolate comprising at least one effector from said set of defined effectors.

7. A method according to any one of claims 1 to 5, further comprising exposing a part of said Solanum plant to a Phytophthora isolate.

8. A method according to any one of claims 1 to 7, wherein said reaction is a hypersensitive response (HR).

9. An isolated R-gene obtainable by the method of any one of claims 4 to 8.

10. An isolated R-gene according to claim 9, wherein said R-gene is isolated from Solanum stoloniferum or Solanum papita.

11. An isolated R-gene according to claim 9 or 10, wherein said R-gene is *Rpi-stol* or *Rpi-pta1.*

12. A method for determining whether an R-gene from Solanum provides resistance to a variety of Phytophthora pathotypes, comprising providing a plant with said R-gene and testing said plant for a reaction to a defined set of effectors, representing said variety of pathotypes.

13. A method for determining whether a set of R-genes from Solanum provides resistance to a variety of Phytophthora pathotypes, comprising providing a plant with said set of R-genes and testing said plant for a reaction to a defined set of effectors, representing said variety of pathotypes.

14. A method for determining whether a set of effectors is representative for a variety of Phytophthora pathotypes, comprising identifying a plant which gives a reaction against said set of effectors and testing whether said plant is resistant against said variety of pathotypes.

15. A method according to any one of claims 12 to 14 , wherein said effector is an elicitor.

16. A method for providing a potato plant with a combination of resistance genes which provides resistance against a variety of pathotypes of Phytophtora, comprising providing said plant with at least two R-genes that together provide resistance against said variety of pathotypes, said R-genes being identifiable/identified by a method according to claim 12.

17. A method according to claim 16, wherein at least one gene of said combination of resistance genes is *Rpi-blb-3* or *Rpi-stol* or *Rpi-pta1.*

18. A method for providing a potato plant having acquired resistance to a variety of Phytophthora pathotypes through the introduction of an R-gene with additional resistance against at least one additional pathotype of Phytophthora, comprising providing said plant with at least one further R-gene obtainable/obtained by a method according to any one of claims 1 to 8.

19. A method according to claim 17, wherein said further R-gene is *Rpi-blb3* or *Rpi-stol* or *Rpi-pta1.*

20. A plant obtainable by the method according to any one of claim 16-19.

21. A method for selecting a plant that is to be used in breeding, comprising exposing a part of a plant to at least one elicitor and determining the presence or absence of a reaction to said at least one elicitor.


SEQUENCE LISTING

<110>  Coöperatie AVEBE U.A.

<120>  Identification, classification and optionally stacking of R-genes
        in Solanum using an effector-receptor approach

<130>  P76980EP00

<140>  EP 07101268
<141>  2007-01-26

<160>  21

<170>  PatentIn version 3.3

<210>  1
<211>  4
<212>  PRT
<213>  Artificial

<220>
<223>  motif


<220>
<221>  VARIANT
<222>  (2)..(2)
<223>  A, C, D, E, F, G, H, I, K, L, M, N, P, Q, R, T, V, W, Y

<400>  1

Arg Ser Leu Arg
1


<210>  2
<211>  5
<212>  PRT
<213>  Artificial

<220>
<223>  Host-cell targeting signal


<220>
<221>  VARIANT
<222>  (2)..(2)
<223>  A, C, D, E, F, G, H, I, K, L, M, N, P, Q, R, T, V, W, Y

<220>
<221>  VARIANT
<222>  (4)..(4)
<223>  A, C, D, E, F, G, H, I, K, L, M, N, P, Q, R, T, V, W, Y

<220>
<221>  VARIANT
<222>  (5)..(5)
<223>  Q

<400>  2

Arg Ser Leu Ser Glu
1               5


<210>  3
<211>  25

<212>  DNA
<213>  Artificial

<220>
<223>  primer GWPBlb1F

<400>  3
caccatgttg taattattgg cgaac                                          25


<210>  4
<211>  24
<212>  DNA
<213>  Artificial

<220>
<223>  primer TBlb1R

<400>  4
gttgttataa gggtataagt gagc                                           24


<210>  5
<211>  396
<212>  DNA
<213>  Phytophthora infestans

<400>  5
gtttcatcca atctcaacac cgccgtgaat tacgcttcca catccaagat tcgctttctg      60

tcgactgagt acaacgccga tgaaaaaaga agcttgcgag gtgactacaa caatgaggtc     120

acaaaagagc ccaacacgtc tgacgaagag cgggcgtttt ctatctcaaa gtctgcggaa     180

tacgtgaaga tggtacttta tggattcaaa cttggatttt ctcctcgcac tcagtccaag     240

acggtgttgc gatacgaaga taaactgttt acggctctct ataaatccgg agagacgccg     300

agaagcctaa ggaccaagca tctcgataag gcttccgcta gcgtattttt caacagattc     360

aaaaaatggt acgataaaaa cgttggccct agctag                               396


<210>  6
<211>  396
<212>  DNA
<213>  Phytophthora infestans

<400>  6
gtttcatcca atctaaacac cgccgtgaat tacgcttcca catccaagat tcgctttctg      60

tcgactgagt acaacgccga tgaaaaaaga agcttgcgag gtgactacaa caatgaggtc     120

acaaaagagc ccaacacgtc tgacgaagag cgggcgtttt ctatctcaaa gtctgcggaa     180

tacgtgaaga tggtacttta tggattcaaa cttggatttt ctcctcgcac tcagtccaag     240

acggtgttgc gatacgaaga taaactgttt acggccctct ataaattagg agagacgccg     300

ataagcctaa ggaccaagca tctcgataag gcttccggta gcgtattttt caacagattc     360

aaaaactggt acgataaaaa cgttggccct agctag                               396


<210>  7
<211>  396
<212>  DNA
<213>  Phytophthora infestans

<400>  7
gtttcatcca atctcaacac cgccgggaat tacgcttcca catccaagat tcgctttctg      60

tcgactgagt acagcgccga tgaaaaaaga agcttgcggg gtgactacaa caatgaggtc     120

acaaaagagc ccaacacggc tgacgaagag cgggcgtttt ctatctcaaa gtctgcggaa     180

tacgtgaaga tggtatttta tgcattcaaa attggatttt ctcctcgcac tcagtccaag     240

acggtgttgc gatacgaaga taaactgttt acggctctct ataaatccgg agagacgccg     300

ataagcctaa ggaccaagta tctcgataag gcttccgcta gcgtattttt caacagattc     360

aaaaactggt acgataaaaa cgttggccct agctag                               396

<210>  8
<211>  396
<212>  DNA
<213>  Phytophthora infestans

<400>  8
gtttcatcca atctcaacac cgccgggaat gacgcttcca catccaagat tcgctttctg      60

tcgactgagt acaacgccga tgaaaaaaga agcttgcggg gtgactacaa caatgaggtc     120

acaaaagagc ccaacacggc tgacgaagag cgggcgtttt ctatctcaaa ctctgtggaa     180

aaagtgaagt tgggattgta tgcattaaag attgcttttt cccctcgcac tcagtccaag     240

acggtgttgc gatacgaaga taaactgttt acgtatctcc ataaatccgg agagacgccg     300

gctagctaca agaacaagca tcccgataag gcttccgctg gcgtattttt caacagattc     360

aaaaactggt acgataaaaa cgttggccct agctag                               396

<210>  9
<211>  132
<212>  PRT
<213>  Phytophthora infestans

<400>  9

Met Val Ser Ser Asn Leu Asn Thr Ala Val Asn Tyr Ala Ser Thr Ser
1               5                   10                  15

Lys Ile Arg Phe Leu Ser Thr Glu Tyr Asn Ala Asp Glu Lys Arg Ser
            20                  25                  30

Leu Arg Gly Asp Tyr Asn Asn Glu Val Thr Lys Glu Pro Asn Thr Ser
        35                  40                  45

Asp Glu Glu Arg Ala Phe Ser Ile Ser Lys Ser Ala Glu Tyr Val Lys
    50                  55                  60

Met Val Leu Tyr Gly Phe Lys Leu Gly Phe Ser Pro Arg Thr Gln Ser
65                  70                  75                  80

Lys Thr Val Leu Arg Tyr Glu Asp Lys Leu Phe Thr Ala Leu Tyr Lys
                85                  90                  95

Ser Gly Glu Thr Pro Arg Ser Leu Arg Thr Lys His Leu Asp Lys Ala
            100                 105                 110

Ser Ala Ser Val Phe Phe Asn Arg Phe Lys Lys Trp Tyr Asp Lys Asn
        115                 120                 125

Val Gly Pro Ser
    130

<210>  10
<211>  132
<212>  PRT
<213>  Phytophthora infestans

<400>  10

Met Val Ser Ser Asn Leu Asn Thr Ala Val Asn Tyr Ala Ser Thr Ser
1               5                   10                  15

Lys Ile Arg Phe Leu Ser Thr Glu Tyr Asn Ala Asp Glu Lys Arg Ser
            20                  25                  30

Leu Arg Gly Asp Tyr Asn Asn Glu Val Thr Lys Glu Pro Asn Thr Ser
        35                  40                  45

Asp Glu Glu Arg Ala Phe Ser Ile Ser Lys Ser Ala Glu Tyr Val Lys
    50                  55                  60

Met Val Leu Tyr Gly Phe Lys Leu Gly Phe Ser Pro Arg Thr Gln Ser
65                  70                  75                  80

Lys Thr Val Leu Arg Tyr Glu Asp Lys Leu Phe Thr Ala Leu Tyr Lys
                85                  90                  95

Ser Gly Glu Thr Pro Arg Ser Leu Arg Thr Lys His Leu Asp Lys Ala
            100                 105                 110

Ser Ala Ser Val Phe Phe Asn Arg Phe Lys Asn Trp Tyr Asp Lys Asn
        115                 120                 125

Val Gly Pro Ser
    130

<210>  11
<211>  132
<212>  PRT
<213>  Phytophthora infestans

<400>  11

Met Val Ser Ser Asn Leu Asn Thr Ala Val Asn Tyr Ala Ser Thr Ser
1               5                   10                  15

Lys Ile Arg Phe Leu Ser Thr Glu Tyr Asn Ala Asp Glu Lys Arg Ser

20                       25                       30

Leu Arg Gly Asp Tyr Asn Asn Glu Val Thr Lys Glu Pro Asn Thr Ser
        35                      40                      45

Asp Glu Glu Arg Ala Phe Ser Ile Ser Lys Ser Ala Glu Tyr Val Lys
    50                      55                      60

Met Val Leu Tyr Gly Phe Lys Leu Gly Phe Ser Pro Arg Thr Gln Ser
65                      70                      75                      80

Lys Thr Val Leu Arg Tyr Glu Asp Lys Leu Phe Thr Ala Leu Tyr Lys
                    85                      90                      95

Leu Gly Glu Thr Pro Ile Ser Leu Arg Thr Lys His Leu Asp Lys Ala
            100                     105                     110

Ser Gly Ser Val Phe Phe Asn Arg Phe Lys Asn Trp Tyr Asp Lys Asn
        115                     120                     125

Val Gly Pro Ser
    130

<210>  12
<211>  132
<212>  PRT
<213>  Phytophthora infestans

<400>  12

Met Val Ser Ser Asn Leu Asn Thr Ala Gly Asn Tyr Ala Ser Thr Ser
1               5                   10                  15

Lys Ile Arg Phe Leu Ser Thr Glu Tyr Ser Ala Asp Glu Lys Arg Ser
            20                  25                  30

Leu Arg Gly Asp Tyr Asn Asn Glu Val Thr Lys Glu Pro Asn Thr Ala
        35                  40                  45

Asp Glu Glu Arg Ala Phe Ser Ile Ser Lys Ser Ala Glu Tyr Val Lys
    50                  55                  60

Met Val Phe Tyr Ala Phe Lys Ile Gly Phe Ser Pro Arg Thr Gln Ser
65                  70                  75                  80

Lys Thr Val Leu Arg Tyr Glu Asp Lys Leu Phe Thr Ala Leu Tyr Lys
                85                  90                  95

Ser Gly Glu Thr Pro Ile Ser Leu Arg Thr Lys Tyr Leu Asp Lys Ala
            100                 105                 110

Ser Ala Ser Val Phe Phe Asn Arg Phe Lys Asn Trp Tyr Asp Lys Asn
        115                 120                 125

Val Gly Pro Ser
    130

<210>  13
<211>  132
<212>  PRT
<213>  Phytophthora infestans

<400>  13

Met Val Ser Ser Asn Leu Asn Thr Ala Gly Asn Asp Ala Ser Thr Ser
1               5                   10                  15

Lys Ile Arg Phe Leu Ser Thr Glu Tyr Asn Ala Asp Glu Lys Arg Ser
            20                  25                  30

Leu Arg Gly Asp Tyr Asn Asn Glu Val Thr Lys Glu Pro Asn Thr Ala
        35                  40                  45

Asp Glu Glu Arg Ala Phe Ser Ile Ser Asn Ser Val Glu Lys Val Lys
    50                  55                  60

Leu Gly Leu Tyr Ala Leu Lys Ile Ala Phe Ser Pro Arg Thr Gln Ser
65                  70                  75                  80

Lys Thr Val Leu Arg Tyr Glu Asp Lys Leu Phe Thr Tyr Leu His Lys
                85                  90                  95

Ser Gly Glu Thr Pro Ala Ser Tyr Lys Asn Lys His Pro Asp Lys Ala
            100                 105                 110

Ser Ala Gly Val Phe Phe Asn Arg Phe Lys Asn Trp Tyr Asp Lys Asn
        115                 120                 125

Val Gly Pro Ser
    130

<210>  14
<211>  2913
<212>  DNA
<213>  Solanum stoloniferum

<220>
<221>  CDS
<222>  (1)..(2913)

<400>  14
atg gct gaa gct ttc att caa gtt ctg tta gac aat ctc act tct ttc       48
Met Ala Glu Ala Phe Ile Gln Val Leu Leu Asp Asn Leu Thr Ser Phe
1               5                   10                  15

ctc aaa ggg gaa ctt aca ttg ctt ttc ggt ttt caa gat gag ttc caa       96
Leu Lys Gly Glu Leu Thr Leu Leu Phe Gly Phe Gln Asp Glu Phe Gln
            20                  25                  30

agg ctt tca agc atg ttt tct aca atc caa gcc gtc ctt gaa gat gct 144
Arg Leu Ser Ser Met Phe Ser Thr Ile Gln Ala Val Leu Glu Asp Ala
        35                  40                  45

cag gag aag caa ctc aac aac aag cct cta gaa aat tgg ttg caa aaa 192
Gln Glu Lys Gln Leu Asn Asn Lys Pro Leu Glu Asn Trp Leu Gln Lys
    50                  55                  60

ctc aat gct gct aca tac gaa gtc gat gac atc ttg gat gaa tat aaa 240
Leu Asn Ala Ala Thr Tyr Glu Val Asp Asp Ile Leu Asp Glu Tyr Lys
65                  70                  75                  80

acc aag gcc aca aga ttc tcc cag tct gaa tat ggc cgt tat cat cca 288
Thr Lys Ala Thr Arg Phe Ser Gln Ser Glu Tyr Gly Arg Tyr His Pro
                85                  90                  95

aag gtt atc cct ttc cgt cac aag gtc ggg aaa agg atg gac caa gtg 336
Lys Val Ile Pro Phe Arg His Lys Val Gly Lys Arg Met Asp Gln Val
            100                 105                 110

atg aaa aaa cta aag gca att gct gag gaa aga aag aat ttt cat ttg 384
Met Lys Lys Leu Lys Ala Ile Ala Glu Glu Arg Lys Asn Phe His Leu
        115                 120                 125

cac gaa aaa att gta gag aga caa gct gtt aga cgg gaa aca ggt tct 432
His Glu Lys Ile Val Glu Arg Gln Ala Val Arg Arg Glu Thr Gly Ser
    130                 135                 140

gta tta acc gaa ccg cag gtt tat gga aga gac aaa gag aaa gat gag 480
Val Leu Thr Glu Pro Gln Val Tyr Gly Arg Asp Lys Glu Lys Asp Glu
145                 150                 155                 160

ata gtg aaa atc cta ata aac aat gtt agt gat gcc caa cac ctt tca 528
Ile Val Lys Ile Leu Ile Asn Asn Val Ser Asp Ala Gln His Leu Ser
                165                 170                 175

gtc ctc cca ata ctt ggt atg ggg gga tta gga aaa acg act ctt gcc 576
Val Leu Pro Ile Leu Gly Met Gly Gly Leu Gly Lys Thr Thr Leu Ala
            180                 185                 190

caa atg gtc ttc aat gac cag aga gtt act gag cat ttc cat tcc aaa 624
Gln Met Val Phe Asn Asp Gln Arg Val Thr Glu His Phe His Ser Lys
        195                 200                 205

ata tgg att tgt gtc tcg gaa gat ttt gat gag aag agg tta ata aag 672
Ile Trp Ile Cys Val Ser Glu Asp Phe Asp Glu Lys Arg Leu Ile Lys
    210                 215                 220

gca att gta gaa tct att gaa gga agg cca cta ctt ggt gag atg gac 720
Ala Ile Val Glu Ser Ile Glu Gly Arg Pro Leu Leu Gly Glu Met Asp
225                 230                 235                 240

ttg gct cca ctt caa aag aag ctt cag gag ttg ctg aat gga aaa aga 768
Leu Ala Pro Leu Gln Lys Lys Leu Gln Glu Leu Leu Asn Gly Lys Arg
                245                 250                 255

tac ttg ctt gtc tta gat gat gtt tgg aat gaa gat caa cag aag tgg 816
Tyr Leu Leu Val Leu Asp Asp Val Trp Asn Glu Asp Gln Gln Lys Trp
            260                 265                 270

gca aat tta aga gca gtc ttg aag gtt gga gca agt ggt gct tct gtt 864
Ala Asn Leu Arg Ala Val Leu Lys Val Gly Ala Ser Gly Ala Ser Val
        275                 280                 285

cta acc act act cgt ctt gaa aag gtt gga tca att atg gga aca ttg 912
Leu Thr Thr Thr Arg Leu Glu Lys Val Gly Ser Ile Met Gly Thr Leu
    290                 295                 300

caa cca tat gaa ctg tca aat ctg tct caa gaa gat tgt tgg ttg ttg 960
Gln Pro Tyr Glu Leu Ser Asn Leu Ser Gln Glu Asp Cys Trp Leu Leu
305                 310                 315                 320

ttc atg caa cgt gca ttt gga cac caa gaa gaa ata aat cca aac ctt 1008
Phe Met Gln Arg Ala Phe Gly His Gln Glu Glu Ile Asn Pro Asn Leu
                325                 330                 335

gtg gca atc gga aag gag att gtg aaa aaa agt ggt ggt gtg cct cta 1056
Val Ala Ile Gly Lys Glu Ile Val Lys Lys Ser Gly Gly Val Pro Leu
            340                 345                 350

gca gcc aaa act ctt gga ggt att ttg tgc ttc aag aga gaa gaa aga 1104
Ala Ala Lys Thr Leu Gly Gly Ile Leu Cys Phe Lys Arg Glu Glu Arg
        355                 360                 365

gca tgg gaa cat gtg aga gac agt ccg att tgg aat ttg cct caa gat 1152
Ala Trp Glu His Val Arg Asp Ser Pro Ile Trp Asn Leu Pro Gln Asp
    370                 375                 380

gaa agt tct att ctg cct gcc ctg agg ctt agt tac cat caa ctt cca 1200
Glu Ser Ser Ile Leu Pro Ala Leu Arg Leu Ser Tyr His Gln Leu Pro
385                 390                 395                 400

ctt gat ttg aaa caa tgc ttt gcg tat tgt gcg gtg ttc cca aag gat 1248
Leu Asp Leu Lys Gln Cys Phe Ala Tyr Cys Ala Val Phe Pro Lys Asp
                405                 410                 415

gcc aaa atg gaa aaa gaa aag cta atc tct ctc tgg atg gcg cat ggt 1296
Ala Lys Met Glu Lys Glu Lys Leu Ile Ser Leu Trp Met Ala His Gly
            420                 425                 430

ttt ctt tta tca aaa gga aac atg gag cta gag gat gtg ggt gat gaa 1344
Phe Leu Leu Ser Lys Gly Asn Met Glu Leu Glu Asp Val Gly Asp Glu
        435                 440                 445

gta tgg aaa gaa tta tac ttg agg tct ttt ttc caa gag att gaa gtt 1392
Val Trp Lys Glu Leu Tyr Leu Arg Ser Phe Phe Gln Glu Ile Glu Val
    450                 455                 460

aaa gat ggt aaa act tat ttc aag atg cat gat ctc atc cat gat ttg 1440
Lys Asp Gly Lys Thr Tyr Phe Lys Met His Asp Leu Ile His Asp Leu
465                 470                 475                 480

gca aca tct ctg ttt tca gca aac aca tca agc agc aat atc cgt gaa 1488
Ala Thr Ser Leu Phe Ser Ala Asn Thr Ser Ser Ser Asn Ile Arg Glu
                485                 490                 495

ata aat aaa cac agt tac aca cat atg atg tcc att ggt ttc gcc gaa 1536
Ile Asn Lys His Ser Tyr Thr His Met Met Ser Ile Gly Phe Ala Glu
            500                 505                 510

gtg gtg ttt ttt tac act ctt ccc ccc ttg gaa aag ttt atc tcg tta 1584
Val Val Phe Phe Tyr Thr Leu Pro Pro Leu Glu Lys Phe Ile Ser Leu
        515                 520                 525

aga gtg ctt aat cta ggt gat tcg aca ttt aat aag tta cca tct tcc 1632
Arg Val Leu Asn Leu Gly Asp Ser Thr Phe Asn Lys Leu Pro Ser Ser
    530                 535                 540

att gga gat cta gta cat tta aga tac ttg aac ctg tat ggc agt ggc 1680
Ile Gly Asp Leu Val His Leu Arg Tyr Leu Asn Leu Tyr Gly Ser Gly
545                 550                 555                 560

atg cgt agt ctt cca aag cag tta tgc aag ctt caa aat ctg caa act 1728
Met Arg Ser Leu Pro Lys Gln Leu Cys Lys Leu Gln Asn Leu Gln Thr
                565                 570                 575

ctt gat cta caa tat tgc acc aag ctt tgt tgt ttg cca aaa gaa aca 1776
Leu Asp Leu Gln Tyr Cys Thr Lys Leu Cys Cys Leu Pro Lys Glu Thr
            580                 585                 590

agt aaa ctt ggt agt ctc cga aat ctt tta ctt gat ggt agc cag tca 1824
Ser Lys Leu Gly Ser Leu Arg Asn Leu Leu Leu Asp Gly Ser Gln Ser
        595                 600                 605

ttg act tgt atg cca cca agg ata gga tca ttg aca tgc ctt aag act 1872
Leu Thr Cys Met Pro Pro Arg Ile Gly Ser Leu Thr Cys Leu Lys Thr
    610                 615                 620

cta ggt caa ttt gtt gtt gga agg aag aaa ggt tat caa ctt ggt gaa 1920
Leu Gly Gln Phe Val Val Gly Arg Lys Lys Gly Tyr Gln Leu Gly Glu
625                 630                 635                 640

cta gga aac cta aat ctc tat ggc tca att aaa atc tcg cat ctt gag 1968
Leu Gly Asn Leu Asn Leu Tyr Gly Ser Ile Lys Ile Ser His Leu Glu
                645                 650                 655

aga gtg aag aat gat aag gac gca aaa gaa gcc aat tta tct gca aaa 2016
Arg Val Lys Asn Asp Lys Asp Ala Lys Glu Ala Asn Leu Ser Ala Lys
            660                 665                 670

ggg aat ctg cat tct tta agc atg agt tgg aat aac ttt gga cca cat 2064
Gly Asn Leu His Ser Leu Ser Met Ser Trp Asn Asn Phe Gly Pro His
        675                 680                 685

ata tat gaa tca gaa gaa gtt aaa gtg ctt gaa gcc ctc aaa cca cac 2112
Ile Tyr Glu Ser Glu Glu Val Lys Val Leu Glu Ala Leu Lys Pro His
    690                 695                 700

tcc aat ctg act tct tta aaa atc tat ggc ttc aga gga atc cat ctc 2160
Ser Asn Leu Thr Ser Leu Lys Ile Tyr Gly Phe Arg Gly Ile His Leu
705                 710                 715                 720

cca gag tgg atg aat cac tca gta ttg aaa aat att gtc tct att cta 2208
Pro Glu Trp Met Asn His Ser Val Leu Lys Asn Ile Val Ser Ile Leu
                725                 730                 735

att agc aac ttc aga aac tgc tca tgc tta cca ccc ttt ggt gat ctg 2256
Ile Ser Asn Phe Arg Asn Cys Ser Cys Leu Pro Pro Phe Gly Asp Leu
            740                 745                 750

cct tgt cta gaa agt cta gag tta cac tgg ggg tct gcg gat gtg gag 2304
Pro Cys Leu Glu Ser Leu Glu Leu His Trp Gly Ser Ala Asp Val Glu
        755                 760                 765

tat gtt gaa gaa gtg gat att gat gtt cat tct gga ttc ccc aca aga 2352
Tyr Val Glu Glu Val Asp Ile Asp Val His Ser Gly Phe Pro Thr Arg
    770                 775                 780

ata agg ttt cca tcc ttg agg aaa ctt gat ata tgg gac ttt ggt agt 2400
Ile Arg Phe Pro Ser Leu Arg Lys Leu Asp Ile Trp Asp Phe Gly Ser
785                 790                 795                 800

ctg aaa gga ttg ctg aaa aag gaa gga gaa gag caa ttc cct gtg ctt 2448
Leu Lys Gly Leu Leu Lys Lys Glu Gly Glu Glu Gln Phe Pro Val Leu
                805                 810                 815

gaa gag atg ata att cac gag tgc cct ttt ctg acc ctt tct tct aat 2496
Glu Glu Met Ile Ile His Glu Cys Pro Phe Leu Thr Leu Ser Ser Asn
            820                 825                 830

ctt agg gct ctt act tcc ctc aga att tgc tat aat aaa gta gct act 2544
Leu Arg Ala Leu Thr Ser Leu Arg Ile Cys Tyr Asn Lys Val Ala Thr
        835                 840                 845

tca ttc cca gaa gag atg ttc aaa aac ctt gca aat ctc aaa tac ttg 2592
Ser Phe Pro Glu Glu Met Phe Lys Asn Leu Ala Asn Leu Lys Tyr Leu
    850                 855                 860

aca atc tct cgg tgc aat aat ctc aaa gag ctg cct acc agc ttg gct 2640
Thr Ile Ser Arg Cys Asn Asn Leu Lys Glu Leu Pro Thr Ser Leu Ala
865                 870                 875                 880

agt ctg aat gct ttg aaa agt cta aaa att caa ttg tgt tgc gca cta 2688
Ser Leu Asn Ala Leu Lys Ser Leu Lys Ile Gln Leu Cys Cys Ala Leu
                885                 890                 895

gag agt ctc cct gag gaa ggg ctg gaa ggt tta tct tca ctc aca gag 2736
Glu Ser Leu Pro Glu Glu Gly Leu Glu Gly Leu Ser Ser Leu Thr Glu
            900                 905                 910

tta ttt gtt gaa cac tgt aac atg cta aaa tgt tta cca gag gga ttg 2784
Leu Phe Val Glu His Cys Asn Met Leu Lys Cys Leu Pro Glu Gly Leu
        915                 920                 925

cag cac cta aca acc ctc aca agt tta aaa att cgg gga tgt cca caa 2832
Gln His Leu Thr Thr Leu Thr Ser Leu Lys Ile Arg Gly Cys Pro Gln
    930                 935                 940

ctg atc aag cgg tgt gag aag gga ata gga gaa gac tgg cac aaa att 2880
Leu Ile Lys Arg Cys Glu Lys Gly Ile Gly Glu Asp Trp His Lys Ile
945                 950                 955                 960

tct cac att cct aat gtg aat ata tat aat taa 2913
Ser His Ile Pro Asn Val Asn Ile Tyr Asn
                965                 970

<210> 15
<211> 970
<212> PRT
<213> Solanum stoloniferum

<400> 15

Met Ala Glu Ala Phe Ile Gln Val Leu Leu Asp Asn Leu Thr Ser Phe
1               5                   10                  15

Leu Lys Gly Glu Leu Thr Leu Leu Phe Gly Phe Gln Asp Glu Phe Gln
            20                  25                  30

Arg Leu Ser Ser Met Phe Ser Thr Ile Gln Ala Val Leu Glu Asp Ala
        35                  40                  45

Gln Glu Lys Gln Leu Asn Asn Lys Pro Leu Glu Asn Trp Leu Gln Lys
    50                  55                  60

Leu Asn Ala Ala Thr Tyr Glu Val Asp Asp Ile Leu Asp Glu Tyr Lys
65                  70                  75                  80

Thr Lys Ala Thr Arg Phe Ser Gln Ser Glu Tyr Gly Arg Tyr His Pro
                85                  90                  95

Lys Val Ile Pro Phe Arg His Lys Val Gly Lys Arg Met Asp Gln Val
            100                 105                 110

Met Lys Lys Leu Lys Ala Ile Ala Glu Glu Arg Lys Asn Phe His Leu
        115                 120                 125

His Glu Lys Ile Val Glu Arg Gln Ala Val Arg Arg Glu Thr Gly Ser
    130                 135                 140

Val Leu Thr Glu Pro Gln Val Tyr Gly Arg Asp Lys Glu Lys Asp Glu
145                 150                 155                 160

Ile Val Lys Ile Leu Ile Asn Asn Val Ser Asp Ala Gln His Leu Ser
                165                 170                 175

Val Leu Pro Ile Leu Gly Met Gly Gly Leu Gly Lys Thr Thr Leu Ala
            180                 185                 190

Gln Met Val Phe Asn Asp Gln Arg Val Thr Glu His Phe His Ser Lys
        195                 200                 205

Ile Trp Ile Cys Val Ser Glu Asp Phe Asp Glu Lys Arg Leu Ile Lys
    210                 215                 220

Ala Ile Val Glu Ser Ile Glu Gly Arg Pro Leu Leu Gly Glu Met Asp
225                 230                 235                 240

Leu Ala Pro Leu Gln Lys Lys Leu Gln Glu Leu Leu Asn Gly Lys Arg
                245                 250                 255

Tyr Leu Leu Val Leu Asp Asp Val Trp Asn Glu Asp Gln Gln Lys Trp
            260                 265                 270

Ala Asn Leu Arg Ala Val Leu Lys Val Gly Ala Ser Gly Ala Ser Val
        275                 280                 285

Leu Thr Thr Thr Arg Leu Glu Lys Val Gly Ser Ile Met Gly Thr Leu
    290                 295                 300

Gln Pro Tyr Glu Leu Ser Asn Leu Ser Gln Glu Asp Cys Trp Leu Leu
305                 310                 315                 320

Phe Met Gln Arg Ala Phe Gly His Gln Glu Glu Ile Asn Pro Asn Leu
                325                 330                 335

Val Ala Ile Gly Lys Glu Ile Val Lys Lys Ser Gly Gly Val Pro Leu
            340                 345                 350

Ala Ala Lys Thr Leu Gly Gly Ile Leu Cys Phe Lys Arg Glu Glu Arg
        355                 360                 365

Ala Trp Glu His Val Arg Asp Ser Pro Ile Trp Asn Leu Pro Gln Asp
    370                 375                 380

Glu Ser Ser Ile Leu Pro Ala Leu Arg Leu Ser Tyr His Gln Leu Pro
385                 390                 395                 400

Leu Asp Leu Lys Gln Cys Phe Ala Tyr Cys Ala Val Phe Pro Lys Asp
                405                 410                 415

Ala Lys Met Glu Lys Glu Lys Leu Ile Ser Leu Trp Met Ala His Gly
            420                 425                 430

Phe Leu Leu Ser Lys Gly Asn Met Glu Leu Glu Asp Val Gly Asp Glu
        435                 440                 445

Val Trp Lys Glu Leu Tyr Leu Arg Ser Phe Phe Gln Glu Ile Glu Val
    450                 455                 460

Lys Asp Gly Lys Thr Tyr Phe Lys Met His Asp Leu Ile His Asp Leu
465                 470                 475                 480

Ala Thr Ser Leu Phe Ser Ala Asn Thr Ser Ser Ser Asn Ile Arg Glu
                485                 490                 495

Ile Asn Lys His Ser Tyr Thr His Met Met Ser Ile Gly Phe Ala Glu
            500                 505                 510

Val Val Phe Phe Tyr Thr Leu Pro Pro Leu Glu Lys Phe Ile Ser Leu
        515                 520                 525

Arg Val Leu Asn Leu Gly Asp Ser Thr Phe Asn Lys Leu Pro Ser Ser
    530                 535                 540

Ile Gly Asp Leu Val His Leu Arg Tyr Leu Asn Leu Tyr Gly Ser Gly
545                 550                 555                 560

Met Arg Ser Leu Pro Lys Gln Leu Cys Lys Leu Gln Asn Leu Gln Thr
                565                 570                 575

Leu Asp Leu Gln Tyr Cys Thr Lys Leu Cys Cys Leu Pro Lys Glu Thr
            580                 585                 590

Ser Lys Leu Gly Ser Leu Arg Asn Leu Leu Leu Asp Gly Ser Gln Ser
        595                 600                 605

Leu Thr Cys Met Pro Pro Arg Ile Gly Ser Leu Thr Cys Leu Lys Thr
    610                 615                 620

Leu Gly Gln Phe Val Val Gly Arg Lys Lys Gly Tyr Gln Leu Gly Glu
625                 630                 635                 640

Leu Gly Asn Leu Asn Leu Tyr Gly Ser Ile Lys Ile Ser His Leu Glu
                645                 650                 655

Arg Val Lys Asn Asp Lys Asp Ala Lys Glu Ala Asn Leu Ser Ala Lys
            660                 665                 670

Gly Asn Leu His Ser Leu Ser Met Ser Trp Asn Asn Phe Gly Pro His
        675                 680                 685

Ile Tyr Glu Ser Glu Glu Val Lys Val Leu Glu Ala Leu Lys Pro His
    690                 695                 700

Ser Asn Leu Thr Ser Leu Lys Ile Tyr Gly Phe Arg Gly Ile His Leu
705                 710                 715                 720

Pro Glu Trp Met Asn His Ser Val Leu Lys Asn Ile Val Ser Ile Leu
                725                 730                 735

Ile Ser Asn Phe Arg Asn Cys Ser Cys Leu Pro Pro Phe Gly Asp Leu
            740                 745                 750

Pro Cys Leu Glu Ser Leu Glu Leu His Trp Gly Ser Ala Asp Val Glu
        755                 760                 765

Tyr Val Glu Glu Val Asp Ile Asp Val His Ser Gly Phe Pro Thr Arg
    770                 775                 780

Ile Arg Phe Pro Ser Leu Arg Lys Leu Asp Ile Trp Asp Phe Gly Ser
785                 790                 795                 800

Leu Lys Gly Leu Leu Lys Lys Glu Gly Glu Glu Gln Phe Pro Val Leu
                805                 810                 815

Glu Glu Met Ile Ile His Glu Cys Pro Phe Leu Thr Leu Ser Ser Asn
            820                 825                 830

Leu Arg Ala Leu Thr Ser Leu Arg Ile Cys Tyr Asn Lys Val Ala Thr
        835                 840                 845

Ser Phe Pro Glu Glu Met Phe Lys Asn Leu Ala Asn Leu Lys Tyr Leu
    850                 855                 860

Thr Ile Ser Arg Cys Asn Asn Leu Lys Glu Leu Pro Thr Ser Leu Ala
865                 870                 875                 880

Ser Leu Asn Ala Leu Lys Ser Leu Lys Ile Gln Leu Cys Cys Ala Leu
                885                 890                 895

Glu Ser Leu Pro Glu Glu Gly Leu Glu Gly Leu Ser Ser Leu Thr Glu
            900                 905                 910

Leu Phe Val Glu His Cys Asn Met Leu Lys Cys Leu Pro Glu Gly Leu
        915                 920                 925

Gln His Leu Thr Thr Leu Thr Ser Leu Lys Ile Arg Gly Cys Pro Gln
    930                 935                 940

Leu Ile Lys Arg Cys Glu Lys Gly Ile Gly Glu Asp Trp His Lys Ile
945                 950                 955                 960

Ser His Ile Pro Asn Val Asn Ile Tyr Asn
                965                 970

<210> 16
<211> 2913
<212> DNA
<213> Solanum papita

<220>
<221> CDS
<222> (1)..(2913)

<400> 16
atg gct gaa gct ttc att caa gtt ctg tta gac aat ctc act tct ttc 48
Met Ala Glu Ala Phe Ile Gln Val Leu Leu Asp Asn Leu Thr Ser Phe
1               5                   10                  15

ctc aaa ggg gaa ctt aca ttg ctt ttc ggt ttt caa gat gag ttc caa 96
Leu Lys Gly Glu Leu Thr Leu Leu Phe Gly Phe Gln Asp Glu Phe Gln
            20                  25                  30

agg ctt tca agc atg ttt tct aca atc caa gcc gtc ctt gaa gat gct 144
Arg Leu Ser Ser Met Phe Ser Thr Ile Gln Ala Val Leu Glu Asp Ala
        35                  40                  45

cag gag aag caa ctc aac aac aag cct cta gaa aat tgg ttg caa aaa 192
Gln Glu Lys Gln Leu Asn Asn Lys Pro Leu Glu Asn Trp Leu Gln Lys
    50                  55                  60

ctc aat gct gct aca tac gaa gtc gat gac atc ttg gat gaa tat aaa 240
Leu Asn Ala Ala Thr Tyr Glu Val Asp Asp Ile Leu Asp Glu Tyr Lys
65                  70                  75                  80

acc aag gcc aca aga ttc tcc cag tct gaa tat ggc cgt tat cat cca 288
Thr Lys Ala Thr Arg Phe Ser Gln Ser Glu Tyr Gly Arg Tyr His Pro
                85                  90                  95

aag gtt atc cct ttc cgt cac aag gtc ggg aaa agg atg gac caa gtg 336
Lys Val Ile Pro Phe Arg His Lys Val Gly Lys Arg Met Asp Gln Val
            100                 105                 110

atg aaa aaa cta aag gca att gct gag gaa aga aag aat ttt cat ttg 384
Met Lys Lys Leu Lys Ala Ile Ala Glu Glu Arg Lys Asn Phe His Leu
        115                 120                 125

cac gaa aaa att gta gag aga caa gct gtt aga cgg gaa aca ggt tct 432
His Glu Lys Ile Val Glu Arg Gln Ala Val Arg Arg Glu Thr Gly Ser
    130                 135                 140

gta tta acc gaa ccg cag gtt tat gga aga gac aaa gag aaa gat gag 480
Val Leu Thr Glu Pro Gln Val Tyr Gly Arg Asp Lys Glu Lys Asp Glu
145                 150                 155                 160

ata gtg aaa atc cta ata aac aat gtt agt gat gcc caa cac ctt tca 528
Ile Val Lys Ile Leu Ile Asn Asn Val Ser Asp Ala Gln His Leu Ser
                165                 170                 175

gtc ctc cca ata ctt ggt atg ggg gga tta gga aaa acg act ctt gcc 576

Val Leu Pro Ile Leu Gly Met Gly Gly Leu Gly Lys Thr Thr Leu Ala
            180                 185                 190

caa atg gtc ttc aat gac cag aga gtt act gag cat ttc cat tcc aaa      624
Gln Met Val Phe Asn Asp Gln Arg Val Thr Glu His Phe His Ser Lys
        195                 200                 205

ata tgg att tgt gtc tcg gaa gat ttt gat gag aag agg tta ata aag      672
Ile Trp Ile Cys Val Ser Glu Asp Phe Asp Glu Lys Arg Leu Ile Lys
    210                 215                 220

gca att gta gaa tct att gaa gga agg cca cta ctt ggt gag atg gac      720
Ala Ile Val Glu Ser Ile Glu Gly Arg Pro Leu Leu Gly Glu Met Asp
225                 230                 235                 240

ttg gct cca ctt caa aag aag ctt cag gag ttg ctg aat gga aaa aga      768
Leu Ala Pro Leu Gln Lys Lys Leu Gln Glu Leu Leu Asn Gly Lys Arg
                245                 250                 255

tac ttg ctt gtc tta gat gat gtt tgg aat gaa gat caa cag aag tgg      816
Tyr Leu Leu Val Leu Asp Asp Val Trp Asn Glu Asp Gln Gln Lys Trp
            260                 265                 270

gct aat tta aga gca gtc ttg aag gtt gga gca agt ggt gct tct gtt      864
Ala Asn Leu Arg Ala Val Leu Lys Val Gly Ala Ser Gly Ala Ser Val
        275                 280                 285

cta acc act act cgt ctt gaa aag gtt gga tca att atg gga aca ttg      912
Leu Thr Thr Thr Arg Leu Glu Lys Val Gly Ser Ile Met Gly Thr Leu
    290                 295                 300

caa cca tat gaa ctg tca aat ctg tct caa gaa gat tgt tgg ttg ttg      960
Gln Pro Tyr Glu Leu Ser Asn Leu Ser Gln Glu Asp Cys Trp Leu Leu
305                 310                 315                 320

ttc atg caa cgt gca ttt gga cac caa gaa gaa ata aat cca aac ctt     1008
Phe Met Gln Arg Ala Phe Gly His Gln Glu Glu Ile Asn Pro Asn Leu
                325                 330                 335

gtg gca atc gga aag gag att gtg aaa aaa agt ggt ggt gtg cct cta     1056
Val Ala Ile Gly Lys Glu Ile Val Lys Lys Ser Gly Gly Val Pro Leu
            340                 345                 350

gca gcc aaa act ctt gga ggt att ttg tgc ttc aag aga gaa gaa aga     1104
Ala Ala Lys Thr Leu Gly Gly Ile Leu Cys Phe Lys Arg Glu Glu Arg
        355                 360                 365

gca tgg gaa cat gtg aga gac agt ccg att tgg aat ttg cct caa gat     1152
Ala Trp Glu His Val Arg Asp Ser Pro Ile Trp Asn Leu Pro Gln Asp
    370                 375                 380

gaa agt tct att ctg cct gcc ctg agg ctt agt tac cat caa ctt cca     1200
Glu Ser Ser Ile Leu Pro Ala Leu Arg Leu Ser Tyr His Gln Leu Pro
385                 390                 395                 400

ctt gat ttg aaa caa tgc ttt gcg tat tgt gcg gtg ttc cca aag gat     1248
Leu Asp Leu Lys Gln Cys Phe Ala Tyr Cys Ala Val Phe Pro Lys Asp
                405                 410                 415

gcc aaa atg gaa aaa gaa aag cta atc tct ctc tgg atg gcg cat ggt     1296
Ala Lys Met Glu Lys Glu Lys Leu Ile Ser Leu Trp Met Ala His Gly
            420                 425                 430

ttt ctt tta tca aaa gga aac atg gag cta gag gat gtg ggc gat gaa     1344
Phe Leu Leu Ser Lys Gly Asn Met Glu Leu Glu Asp Val Gly Asp Glu
        435                 440                 445

gta tgg aaa gaa tta tac ttg agg tct ttt ttc caa gag att gaa gtt     1392

Val Trp Lys Glu Leu Tyr Leu Arg Ser Phe Phe Gln Glu Ile Glu Val
    450                 455                 460

aaa gat ggt aaa act tat ttc aag atg cat gat ctc atc cat gat ttg      1440
Lys Asp Gly Lys Thr Tyr Phe Lys Met His Asp Leu Ile His Asp Leu
465                 470                 475                 480

gca aca tct ctg ttt tca gca aac aca tca agc agc aat atc cgt gaa      1488
Ala Thr Ser Leu Phe Ser Ala Asn Thr Ser Ser Ser Asn Ile Arg Glu
                485                 490                 495

ata aat aaa cac agt tac aca cat atg atg tcc att ggt ttc gcc gaa      1536
Ile Asn Lys His Ser Tyr Thr His Met Met Ser Ile Gly Phe Ala Glu
            500                 505                 510

gtg gtg ttt ttt tac act ctt ccc ccc ttg gaa aag ttt atc tcg tta      1584
Val Val Phe Phe Tyr Thr Leu Pro Pro Leu Glu Lys Phe Ile Ser Leu
        515                 520                 525

aga gtg ctt aat cta ggt gat tcg aca ttt aat aag tta cca tct tcc      1632
Arg Val Leu Asn Leu Gly Asp Ser Thr Phe Asn Lys Leu Pro Ser Ser
    530                 535                 540

att gga gat cta gta cat tta aga tac ttg aac ctg tat ggc agt ggc      1680
Ile Gly Asp Leu Val His Leu Arg Tyr Leu Asn Leu Tyr Gly Ser Gly
545                 550                 555                 560

atg cgt agt ctt cca aag cag tta tgc aag ctt caa aat ctg caa act      1728
Met Arg Ser Leu Pro Lys Gln Leu Cys Lys Leu Gln Asn Leu Gln Thr
                565                 570                 575

ctt gat cta caa tat tgc acc aag ctt tgt tgt ttg cca aaa gaa aca      1776
Leu Asp Leu Gln Tyr Cys Thr Lys Leu Cys Cys Leu Pro Lys Glu Thr
            580                 585                 590

agt aaa ctt ggt agt ctc cga aat ctt tta ctt gat ggt agc cag tca      1824
Ser Lys Leu Gly Ser Leu Arg Asn Leu Leu Leu Asp Gly Ser Gln Ser
        595                 600                 605

ttg act tgt atg cca cca agg ata gga tca ttg aca tgc ctt aag act      1872
Leu Thr Cys Met Pro Pro Arg Ile Gly Ser Leu Thr Cys Leu Lys Thr
    610                 615                 620

cta ggt caa ttt gtt gtt gga agg aag aaa ggt tat caa ctt ggt gaa      1920
Leu Gly Gln Phe Val Val Gly Arg Lys Lys Gly Tyr Gln Leu Gly Glu
625                 630                 635                 640

cta gga aac cta aat ctc tat ggc tca att aaa atc tcg cat ctt gag      1968
Leu Gly Asn Leu Asn Leu Tyr Gly Ser Ile Lys Ile Ser His Leu Glu
                645                 650                 655

aga gtg aag aat gat agg gac gca aaa gaa gcc aat tta tct gca aaa      2016
Arg Val Lys Asn Asp Arg Asp Ala Lys Glu Ala Asn Leu Ser Ala Lys
            660                 665                 670

ggg aat ctg cat tct tta agc atg agt tgg aat aac ttt gga cca cat      2064
Gly Asn Leu His Ser Leu Ser Met Ser Trp Asn Asn Phe Gly Pro His
        675                 680                 685

ata tat gaa tca gaa gaa gtt aaa gtg ctt gaa gcc ctc aaa cca cac      2112
Ile Tyr Glu Ser Glu Glu Val Lys Val Leu Glu Ala Leu Lys Pro His
    690                 695                 700

tcc aat ctg act tct tta aaa atc tat ggc ttc aga gga atc cat ctc      2160
Ser Asn Leu Thr Ser Leu Lys Ile Tyr Gly Phe Arg Gly Ile His Leu
705                 710                 715                 720

cca gag tgg atg aat cac tca gta ttg aaa aat att gtc tct att cta      2208

Pro Glu Trp Met Asn His Ser Val Leu Lys Asn Ile Val Ser Ile Leu
                725                 730                 735

att agc aac ttc aga aac tgc tca tgc tta cca ccc ttt ggt gat ctg      2256
Ile Ser Asn Phe Arg Asn Cys Ser Cys Leu Pro Pro Phe Gly Asp Leu
            740                 745                 750

cct tgt cta gaa agt cta gag tta cac tgg ggg tct gcg gat gtg gag      2304
Pro Cys Leu Glu Ser Leu Glu Leu His Trp Gly Ser Ala Asp Val Glu
        755                 760                 765

tat gtt gaa gaa gtg gat att gat gtt cat tct gga ttc ccc aca aga      2352
Tyr Val Glu Glu Val Asp Ile Asp Val His Ser Gly Phe Pro Thr Arg
    770                 775                 780

ata agg ttt cca tcc ttg agg aaa ctt gat ata tgg gac ttt ggt agt      2400
Ile Arg Phe Pro Ser Leu Arg Lys Leu Asp Ile Trp Asp Phe Gly Ser
785                 790                 795                 800

ctg aaa gga ttg ctg aaa aag gaa gga gaa gag caa ttc cct gtg ctt      2448
Leu Lys Gly Leu Leu Lys Lys Glu Gly Glu Glu Gln Phe Pro Val Leu
                805                 810                 815

gaa gag ctg ata att cac gag tgc cct ttt ctg acc ctt tct tct aat      2496
Glu Glu Leu Ile Ile His Glu Cys Pro Phe Leu Thr Leu Ser Ser Asn
            820                 825                 830

ctt agg gct ctt act tcc ctc aga att tgc tat aat aaa gta gct act      2544
Leu Arg Ala Leu Thr Ser Leu Arg Ile Cys Tyr Asn Lys Val Ala Thr
        835                 840                 845

tca ttc cca gaa gag atg ttc aaa aac ctt gca aat ctc aaa tac ttg      2592
Ser Phe Pro Glu Glu Met Phe Lys Asn Leu Ala Asn Leu Lys Tyr Leu
    850                 855                 860

aca atc tct cgg tgc aat aat ctc aaa gag ctg cct acc agc ttg gct      2640
Thr Ile Ser Arg Cys Asn Asn Leu Lys Glu Leu Pro Thr Ser Leu Ala
865                 870                 875                 880

agt ctg aat gct ttg aaa agt cta aaa att caa ttg tgt tgc gca cta      2688
Ser Leu Asn Ala Leu Lys Ser Leu Lys Ile Gln Leu Cys Cys Ala Leu
                885                 890                 895

gag agt ctc cct gag gaa ggg ctg gaa ggt tta tct tca ctc aca gag      2736
Glu Ser Leu Pro Glu Glu Gly Leu Glu Gly Leu Ser Ser Leu Thr Glu
            900                 905                 910

tta ttt gtt gaa cac tgt aac atg cta aaa tgt tta cca gag gga ttg      2784
Leu Phe Val Glu His Cys Asn Met Leu Lys Cys Leu Pro Glu Gly Leu
        915                 920                 925

cag cac cta aca acc ctc aca agt tta aaa att cgg gga tgt cca caa      2832
Gln His Leu Thr Thr Leu Thr Ser Leu Lys Ile Arg Gly Cys Pro Gln
    930                 935                 940

ctg atc aag cgg tgt gag aag gga ata gga gaa gac tgg cac aaa att      2880
Leu Ile Lys Arg Cys Glu Lys Gly Ile Gly Glu Asp Trp His Lys Ile
945                 950                 955                 960

tct cac att cct aat gtg aat ata tat aat taa                          2913
Ser His Ile Pro Asn Val Asn Ile Tyr Asn
                965                 970

<210>  17
<211>  970
<212>  PRT
<213>  Solanum papita

<400>  17

Met Ala Glu Ala Phe Ile Gln Val Leu Leu Asp Asn Leu Thr Ser Phe
1               5                   10                  15

Leu Lys Gly Glu Leu Thr Leu Leu Phe Gly Phe Gln Asp Glu Phe Gln
            20                  25                  30

Arg Leu Ser Ser Met Phe Ser Thr Ile Gln Ala Val Leu Glu Asp Ala
        35                  40                  45

Gln Glu Lys Gln Leu Asn Asn Lys Pro Leu Glu Asn Trp Leu Gln Lys
    50                  55                  60

Leu Asn Ala Ala Thr Tyr Glu Val Asp Asp Ile Leu Asp Glu Tyr Lys
65                  70                  75                  80

Thr Lys Ala Thr Arg Phe Ser Gln Ser Glu Tyr Gly Arg Tyr His Pro
                85                  90                  95

Lys Val Ile Pro Phe Arg His Lys Val Gly Lys Arg Met Asp Gln Val
            100                 105                 110

Met Lys Lys Leu Lys Ala Ile Ala Glu Glu Arg Lys Asn Phe His Leu
        115                 120                 125

His Glu Lys Ile Val Glu Arg Gln Ala Val Arg Arg Glu Thr Gly Ser
    130                 135                 140

Val Leu Thr Glu Pro Gln Val Tyr Gly Arg Asp Lys Glu Lys Asp Glu
145                 150                 155                 160

Ile Val Lys Ile Leu Ile Asn Asn Val Ser Asp Ala Gln His Leu Ser
                165                 170                 175

Val Leu Pro Ile Leu Gly Met Gly Gly Leu Gly Lys Thr Thr Leu Ala
            180                 185                 190

Gln Met Val Phe Asn Asp Gln Arg Val Thr Glu His Phe His Ser Lys
        195                 200                 205

Ile Trp Ile Cys Val Ser Glu Asp Phe Asp Glu Lys Arg Leu Ile Lys
    210                 215                 220

Ala Ile Val Glu Ser Ile Glu Gly Arg Pro Leu Leu Gly Glu Met Asp
225                 230                 235                 240

Leu Ala Pro Leu Gln Lys Lys Leu Gln Glu Leu Leu Asn Gly Lys Arg
                245                 250                 255

Tyr Leu Leu Val Leu Asp Asp Val Trp Asn Glu Asp Gln Gln Lys Trp

            260                 265                 270

Ala Asn Leu Arg Ala Val Leu Lys Val Gly Ala Ser Gly Ala Ser Val
        275                 280                 285

Leu Thr Thr Thr Arg Leu Glu Lys Val Gly Ser Ile Met Gly Thr Leu
    290                 295                 300

Gln Pro Tyr Glu Leu Ser Asn Leu Ser Gln Glu Asp Cys Trp Leu Leu
305                 310                 315                 320

Phe Met Gln Arg Ala Phe Gly His Gln Glu Glu Ile Asn Pro Asn Leu
                325                 330                 335

Val Ala Ile Gly Lys Glu Ile Val Lys Lys Ser Gly Gly Val Pro Leu
            340                 345                 350

Ala Ala Lys Thr Leu Gly Gly Ile Leu Cys Phe Lys Arg Glu Glu Arg
        355                 360                 365

Ala Trp Glu His Val Arg Asp Ser Pro Ile Trp Asn Leu Pro Gln Asp
    370                 375                 380

Glu Ser Ser Ile Leu Pro Ala Leu Arg Leu Ser Tyr His Gln Leu Pro
385                 390                 395                 400

Leu Asp Leu Lys Gln Cys Phe Ala Tyr Cys Ala Val Phe Pro Lys Asp
                405                 410                 415

Ala Lys Met Glu Lys Glu Lys Leu Ile Ser Leu Trp Met Ala His Gly
            420                 425                 430

Phe Leu Leu Ser Lys Gly Asn Met Glu Leu Glu Asp Val Gly Asp Glu
        435                 440                 445

Val Trp Lys Glu Leu Tyr Leu Arg Ser Phe Phe Gln Glu Ile Glu Val
    450                 455                 460

Lys Asp Gly Lys Thr Tyr Phe Lys Met His Asp Leu Ile His Asp Leu
465                 470                 475                 480

Ala Thr Ser Leu Phe Ser Ala Asn Thr Ser Ser Ser Asn Ile Arg Glu
                485                 490                 495

Ile Asn Lys His Ser Tyr Thr His Met Met Ser Ile Gly Phe Ala Glu
            500                 505                 510

Val Val Phe Phe Tyr Thr Leu Pro Pro Leu Glu Lys Phe Ile Ser Leu
        515                 520                 525

Arg Val Leu Asn Leu Gly Asp Ser Thr Phe Asn Lys Leu Pro Ser Ser

530                 535                 540

Ile Gly Asp Leu Val His Leu Arg Tyr Leu Asn Leu Tyr Gly Ser Gly
545                 550                 555                 560

Met Arg Ser Leu Pro Lys Gln Leu Cys Lys Leu Gln Asn Leu Gln Thr
                565                 570                 575

Leu Asp Leu Gln Tyr Cys Thr Lys Leu Cys Cys Leu Pro Lys Glu Thr
            580                 585                 590

Ser Lys Leu Gly Ser Leu Arg Asn Leu Leu Leu Asp Gly Ser Gln Ser
        595                 600                 605

Leu Thr Cys Met Pro Pro Arg Ile Gly Ser Leu Thr Cys Leu Lys Thr
    610                 615                 620

Leu Gly Gln Phe Val Val Gly Arg Lys Lys Gly Tyr Gln Leu Gly Glu
625                 630                 635                 640

Leu Gly Asn Leu Asn Leu Tyr Gly Ser Ile Lys Ile Ser His Leu Glu
                645                 650                 655

Arg Val Lys Asn Asp Arg Asp Ala Lys Glu Ala Asn Leu Ser Ala Lys
            660                 665                 670

Gly Asn Leu His Ser Leu Ser Met Ser Trp Asn Asn Phe Gly Pro His
        675                 680                 685

Ile Tyr Glu Ser Glu Glu Val Lys Val Leu Glu Ala Leu Lys Pro His
    690                 695                 700

Ser Asn Leu Thr Ser Leu Lys Ile Tyr Gly Phe Arg Gly Ile His Leu
705                 710                 715                 720

Pro Glu Trp Met Asn His Ser Val Leu Lys Asn Ile Val Ser Ile Leu
                725                 730                 735

Ile Ser Asn Phe Arg Asn Cys Ser Cys Leu Pro Pro Phe Gly Asp Leu
            740                 745                 750

Pro Cys Leu Glu Ser Leu Glu Leu His Trp Gly Ser Ala Asp Val Glu
        755                 760                 765

Tyr Val Glu Glu Val Asp Ile Asp Val His Ser Gly Phe Pro Thr Arg
    770                 775                 780

Ile Arg Phe Pro Ser Leu Arg Lys Leu Asp Ile Trp Asp Phe Gly Ser
785                 790                 795                 800

Leu Lys Gly Leu Leu Lys Lys Glu Gly Glu Glu Gln Phe Pro Val Leu

805                 810                 815

Glu Glu Leu Ile Ile His Glu Cys Pro Phe Leu Thr Leu Ser Ser Asn
            820                 825                 830

Leu Arg Ala Leu Thr Ser Leu Arg Ile Cys Tyr Asn Lys Val Ala Thr
        835                 840                 845

Ser Phe Pro Glu Glu Met Phe Lys Asn Leu Ala Asn Leu Lys Tyr Leu
    850                 855                 860

Thr Ile Ser Arg Cys Asn Asn Leu Lys Glu Leu Pro Thr Ser Leu Ala
865                 870                 875                 880

Ser Leu Asn Ala Leu Lys Ser Leu Lys Ile Gln Leu Cys Cys Ala Leu
                885                 890                 895

Glu Ser Leu Pro Glu Glu Gly Leu Glu Gly Leu Ser Ser Leu Thr Glu
            900                 905                 910

Leu Phe Val Glu His Cys Asn Met Leu Lys Cys Leu Pro Glu Gly Leu
        915                 920                 925

Gln His Leu Thr Thr Leu Thr Ser Leu Lys Ile Arg Gly Cys Pro Gln
    930                 935                 940

Leu Ile Lys Arg Cys Glu Lys Gly Ile Gly Glu Asp Trp His Lys Ile
945                 950                 955                 960

Ser His Ile Pro Asn Val Asn Ile Tyr Asn
                965                 970

<210>  18
<211>  2913
<212>  DNA
<213>  Solanum bulbocastanum

<220>
<221>  CDS
<222>  (1)..(2913)

<400>  18
atg gct gaa gct ttc att caa gtt ctg cta gac aat ctc act tct ttc      48
Met Ala Glu Ala Phe Ile Gln Val Leu Leu Asp Asn Leu Thr Ser Phe
1               5                   10                  15

ctc aaa ggg gaa ctt gta ttg ctt ttc ggt ttt caa gat gag ttc caa      96
Leu Lys Gly Glu Leu Val Leu Leu Phe Gly Phe Gln Asp Glu Phe Gln
            20                  25                  30

agg ctt tca agc atg ttt tct aca att caa gcc gtc ctt gaa gat gct      144
Arg Leu Ser Ser Met Phe Ser Thr Ile Gln Ala Val Leu Glu Asp Ala
        35                  40                  45

cag gag aag caa ctc aac aac aag cct cta gaa aat tgg ttg caa aaa      192
Gln Glu Lys Gln Leu Asn Asn Lys Pro Leu Glu Asn Trp Leu Gln Lys

50                  55                  60

ctc aat gct gct aca tat gaa gtc gat gac atc ttg gat gaa tat aaa      240
Leu Asn Ala Ala Thr Tyr Glu Val Asp Asp Ile Leu Asp Glu Tyr Lys
65                  70                  75                  80

acc aag gcc aca aga ttc tcc cag tct gaa tat ggc cgt tat cat cca      288
Thr Lys Ala Thr Arg Phe Ser Gln Ser Glu Tyr Gly Arg Tyr His Pro
                85                  90                  95

aag gtt atc cct ttc cgt cac aag gtc ggg aaa agg atg gac caa gtg      336
Lys Val Ile Pro Phe Arg His Lys Val Gly Lys Arg Met Asp Gln Val
            100                 105                 110

atg aaa aaa cta aag gca att gct gag gaa aga aag aat ttt cat ttg      384
Met Lys Lys Leu Lys Ala Ile Ala Glu Glu Arg Lys Asn Phe His Leu
        115                 120                 125

cac gaa aaa att gta gag aga caa gct gtt aga cgg gaa aca ggt tct      432
His Glu Lys Ile Val Glu Arg Gln Ala Val Arg Arg Glu Thr Gly Ser
    130                 135                 140

gta tta acc gaa ccg cag gtt tat gga aga gac aaa gag aaa gat gag      480
Val Leu Thr Glu Pro Gln Val Tyr Gly Arg Asp Lys Glu Lys Asp Glu
145                 150                 155                 160

ata gtg aaa atc cta ata aac aat gtt agt gat gcc caa cac ctt tca      528
Ile Val Lys Ile Leu Ile Asn Asn Val Ser Asp Ala Gln His Leu Ser
                165                 170                 175

gtc ctc cca ata ctt ggt atg ggg gga tta gga aaa acg act ctt gcc      576
Val Leu Pro Ile Leu Gly Met Gly Gly Leu Gly Lys Thr Thr Leu Ala
            180                 185                 190

caa atg gtc ttc aat gac cag aga gtt act gag cat ttc cat tcc aaa      624
Gln Met Val Phe Asn Asp Gln Arg Val Thr Glu His Phe His Ser Lys
        195                 200                 205

ata tgg att tgt gtc tcg gaa gat ttt gat gag aag agg tta ata aag      672
Ile Trp Ile Cys Val Ser Glu Asp Phe Asp Glu Lys Arg Leu Ile Lys
    210                 215                 220

gca att gta gaa tct att gaa gga agg cca cta ctt ggt gag atg gac      720
Ala Ile Val Glu Ser Ile Glu Gly Arg Pro Leu Leu Gly Glu Met Asp
225                 230                 235                 240

ttg gct cca ctt caa aag aag ctt cag gag ttg ctg aat gga aaa aga      768
Leu Ala Pro Leu Gln Lys Lys Leu Gln Glu Leu Leu Asn Gly Lys Arg
                245                 250                 255

tac ttg ctt gtc tta gat gat gtt tgg aat gaa gat caa cag aag tgg      816
Tyr Leu Leu Val Leu Asp Asp Val Trp Asn Glu Asp Gln Gln Lys Trp
            260                 265                 270

gct aat tta aga gca gtc ttg aag gtt gga gca agt ggt gct tct gtt      864
Ala Asn Leu Arg Ala Val Leu Lys Val Gly Ala Ser Gly Ala Ser Val
        275                 280                 285

cta acc act act cgt ctt gaa aag gtt gga tca att atg gga aca ttg      912
Leu Thr Thr Thr Arg Leu Glu Lys Val Gly Ser Ile Met Gly Thr Leu
    290                 295                 300

caa cca tat gaa ctg tca aat ctg tct caa gaa gat tgt tgg ttg ttg      960
Gln Pro Tyr Glu Leu Ser Asn Leu Ser Gln Glu Asp Cys Trp Leu Leu
305                 310                 315                 320

ttc atg caa cgt gca ttt gga cac caa gaa gaa ata aat cca aac ctt     1008
Phe Met Gln Arg Ala Phe Gly His Gln Glu Glu Ile Asn Pro Asn Leu

                325                 330                 335

gtg gca atc gga aag gag att gtg aaa aaa agt ggt ggt gtg cct cta 1056
Val Ala Ile Gly Lys Glu Ile Val Lys Lys Ser Gly Gly Val Pro Leu
            340                 345                 350

gca gcc aaa act ctt gga ggt att ttg tgc ttc aag aga gaa gaa aga 1104
Ala Ala Lys Thr Leu Gly Gly Ile Leu Cys Phe Lys Arg Glu Glu Arg
        355                 360                 365

gca tgg gaa cat gtg aga gac agt ccg att tgg aat ttg cct caa gat 1152
Ala Trp Glu His Val Arg Asp Ser Pro Ile Trp Asn Leu Pro Gln Asp
    370                 375                 380

gaa agt tct att ctg cct gcc ctg agg ctt agt tac cat caa ctt cca 1200
Glu Ser Ser Ile Leu Pro Ala Leu Arg Leu Ser Tyr His Gln Leu Pro
385                 390                 395                 400

ctt gat ttg aaa caa tgc ttt gcg tat tgt gcg gtg ttc cca aag gat 1248
Leu Asp Leu Lys Gln Cys Phe Ala Tyr Cys Ala Val Phe Pro Lys Asp
                405                 410                 415

gcc aaa atg gaa aaa gaa aag cta atc tct ctc tgg atg gcg cat ggt 1296
Ala Lys Met Glu Lys Glu Lys Leu Ile Ser Leu Trp Met Ala His Gly
            420                 425                 430

ttt ctt tta tca aaa gga aac atg gag cta gag gat gtg ggc gat gaa 1344
Phe Leu Leu Ser Lys Gly Asn Met Glu Leu Glu Asp Val Gly Asp Glu
        435                 440                 445

gta tgg aaa gaa tta tac ttg agg tct ttt ttc caa gag att gaa gtt 1392
Val Trp Lys Glu Leu Tyr Leu Arg Ser Phe Phe Gln Glu Ile Glu Val
    450                 455                 460

aaa gat ggt aaa act tat ttc aag atg cat gat ctc atc cat gat ttg 1440
Lys Asp Gly Lys Thr Tyr Phe Lys Met His Asp Leu Ile His Asp Leu
465                 470                 475                 480

gca aca tct ctg ttt tca gca aac aca tca agc agc aat atc cgt gaa 1488
Ala Thr Ser Leu Phe Ser Ala Asn Thr Ser Ser Ser Asn Ile Arg Glu
                485                 490                 495

ata aat aaa cac agt tac aca cat atg atg tcc att ggt ttc gcc gaa 1536
Ile Asn Lys His Ser Tyr Thr His Met Met Ser Ile Gly Phe Ala Glu
            500                 505                 510

gtg gtg ttt ttt tac act ctt ccc ccc ttg gaa aag ttt atc tcg tta 1584
Val Val Phe Phe Tyr Thr Leu Pro Pro Leu Glu Lys Phe Ile Ser Leu
        515                 520                 525

aga gtg ctt aat cta ggt gat tcg aca ttt aat aag tta cca tct tcc 1632
Arg Val Leu Asn Leu Gly Asp Ser Thr Phe Asn Lys Leu Pro Ser Ser
    530                 535                 540

att gga gat cta gta cat tta aga tac ttg aac ctg tat ggc agt ggc 1680
Ile Gly Asp Leu Val His Leu Arg Tyr Leu Asn Leu Tyr Gly Ser Gly
545                 550                 555                 560

atg cgt agt ctt cca aag cag tta tgc aag ctt caa aat ctg caa act 1728
Met Arg Ser Leu Pro Lys Gln Leu Cys Lys Leu Gln Asn Leu Gln Thr
                565                 570                 575

ctt gat cta caa tat tgc acc aag ctt tgt tgt ttg cca aaa gaa aca 1776
Leu Asp Leu Gln Tyr Cys Thr Lys Leu Cys Cys Leu Pro Lys Glu Thr
            580                 585                 590

agt aaa ctt ggt agt ctc cga aat ctt tta ctt gat ggt agc cag tca 1824
Ser Lys Leu Gly Ser Leu Arg Asn Leu Leu Leu Asp Gly Ser Gln Ser

        595                 600                 605

ttg act tgt atg cca cca agg ata gga tca ttg aca tgc ctt aag act 1872
Leu Thr Cys Met Pro Pro Arg Ile Gly Ser Leu Thr Cys Leu Lys Thr
    610                 615                 620

cta ggt caa ttt gtt gtt gga agg aag aaa ggt tat caa ctt ggt gaa 1920
Leu Gly Gln Phe Val Val Gly Arg Lys Lys Gly Tyr Gln Leu Gly Glu
625                 630                 635                 640

cta gga aac cta aat ctc tat ggc tca att aaa atc tcg cat ctt gag 1968
Leu Gly Asn Leu Asn Leu Tyr Gly Ser Ile Lys Ile Ser His Leu Glu
                645                 650                 655

aga gtg aag aat gat aag gac gca aaa gaa gcc aat tta tct gca aaa 2016
Arg Val Lys Asn Asp Lys Asp Ala Lys Glu Ala Asn Leu Ser Ala Lys
            660                 665                 670

ggg aat ctg cat tct tta agc atg agt tgg aat aac ttt gga cca cat 2064
Gly Asn Leu His Ser Leu Ser Met Ser Trp Asn Asn Phe Gly Pro His
        675                 680                 685

ata tat gaa tca gaa gaa gtt aaa gtg ctt gaa gcc ctc aaa cca cac 2112
Ile Tyr Glu Ser Glu Glu Val Lys Val Leu Glu Ala Leu Lys Pro His
    690                 695                 700

tcc aat ctg act tct tta aaa atc tat ggc ttc aga gga atc cat ctc 2160
Ser Asn Leu Thr Ser Leu Lys Ile Tyr Gly Phe Arg Gly Ile His Leu
705                 710                 715                 720

cca gag tgg atg aat cac tca gta ttg aaa aat att gtc tct att cta 2208
Pro Glu Trp Met Asn His Ser Val Leu Lys Asn Ile Val Ser Ile Leu
                725                 730                 735

att agc aac ttc aga aac tgc tca tgc tta cca ccc ttt ggt gat ctg 2256
Ile Ser Asn Phe Arg Asn Cys Ser Cys Leu Pro Pro Phe Gly Asp Leu
            740                 745                 750

cct tgt cta gaa agt cta gag tta cac tgg ggg tct gcg gat gtg gag 2304
Pro Cys Leu Glu Ser Leu Glu Leu His Trp Gly Ser Ala Asp Val Glu
        755                 760                 765

tat gtt gaa gaa gtg gat att gat gtt cat tct gga ttc ccc aca aga 2352
Tyr Val Glu Glu Val Asp Ile Asp Val His Ser Gly Phe Pro Thr Arg
    770                 775                 780

ata agg ttt cca tcc ttg agg aaa ctt gat ata tgg gac ttt ggt agt 2400
Ile Arg Phe Pro Ser Leu Arg Lys Leu Asp Ile Trp Asp Phe Gly Ser
785                 790                 795                 800

ctg aaa gga ttg ctg aaa aag gaa gga gaa gag caa ttc cct gtg ctt 2448
Leu Lys Gly Leu Leu Lys Lys Glu Gly Glu Glu Gln Phe Pro Val Leu
                805                 810                 815

gaa gag atg ata att cac gag tgc cct ttt ctg acc ctt tct tct aat 2496
Glu Glu Met Ile Ile His Glu Cys Pro Phe Leu Thr Leu Ser Ser Asn
            820                 825                 830

ctt agg gct ctt act tcc ctc aga att tgc tat aat aaa gta gct act 2544
Leu Arg Ala Leu Thr Ser Leu Arg Ile Cys Tyr Asn Lys Val Ala Thr
        835                 840                 845

tca ttc cca gaa gag atg ttc aaa aac ctt gca aat ctc aaa tac ttg 2592
Ser Phe Pro Glu Glu Met Phe Lys Asn Leu Ala Asn Leu Lys Tyr Leu
    850                 855                 860

aca atc tct cgg tgc aat aat ctc aaa gag ctg cct acc agc ttg gct 2640
Thr Ile Ser Arg Cys Asn Asn Leu Lys Glu Leu Pro Thr Ser Leu Ala

865                 870                 875                 880

agt ctg aat gct ttg aaa agt cta aaa att caa ttg tgt tgc gca cta      2688
Ser Leu Asn Ala Leu Lys Ser Leu Lys Ile Gln Leu Cys Cys Ala Leu
                885                 890                 895

gag agt ctc cct gag gaa ggg ctg gaa ggt tta tct tca ctc aca gag      2736
Glu Ser Leu Pro Glu Glu Gly Leu Glu Gly Leu Ser Ser Leu Thr Glu
            900                 905                 910

tta ttt gtt gaa cac tgt aac atg cta aaa tgt tta cca gag gga ttg      2784
Leu Phe Val Glu His Cys Asn Met Leu Lys Cys Leu Pro Glu Gly Leu
        915                 920                 925

cag cac cta aca acc ctc aca agt tta aaa att cgg gga tgt cca caa      2832
Gln His Leu Thr Thr Leu Thr Ser Leu Lys Ile Arg Gly Cys Pro Gln
    930                 935                 940

ctg atc aag cgg tgt gag aag gga ata gga gaa gac tgg cac aaa att      2880
Leu Ile Lys Arg Cys Glu Lys Gly Ile Gly Glu Asp Trp His Lys Ile
945                 950                 955                 960

tct cac att cct aat gtg aat ata tat att taa                          2913
Ser His Ile Pro Asn Val Asn Ile Tyr Ile
                965                 970


<210>  19
<211>  970
<212>  PRT
<213>  Solanum bulbocastanum

<400>  19

Met Ala Glu Ala Phe Ile Gln Val Leu Leu Asp Asn Leu Thr Ser Phe
1               5                   10                  15

Leu Lys Gly Glu Leu Val Leu Leu Phe Gly Phe Gln Asp Glu Phe Gln
            20                  25                  30

Arg Leu Ser Ser Met Phe Ser Thr Ile Gln Ala Val Leu Glu Asp Ala
        35                  40                  45

Gln Glu Lys Gln Leu Asn Asn Lys Pro Leu Glu Asn Trp Leu Gln Lys
    50                  55                  60

Leu Asn Ala Ala Thr Tyr Glu Val Asp Asp Ile Leu Asp Glu Tyr Lys
65                  70                  75                  80

Thr Lys Ala Thr Arg Phe Ser Gln Ser Glu Tyr Gly Arg Tyr His Pro
                85                  90                  95

Lys Val Ile Pro Phe Arg His Lys Val Gly Lys Arg Met Asp Gln Val
            100                 105                 110

Met Lys Lys Leu Lys Ala Ile Ala Glu Glu Arg Lys Asn Phe His Leu
        115                 120                 125

His Glu Lys Ile Val Glu Arg Gln Ala Val Arg Arg Glu Thr Gly Ser
    130                 135                 140

Val Leu Thr Glu Pro Gln Val Tyr Gly Arg Asp Lys Glu Lys Asp Glu
145                 150                 155                 160

Ile Val Lys Ile Leu Ile Asn Asn Val Ser Asp Ala Gln His Leu Ser
                165                 170                 175

Val Leu Pro Ile Leu Gly Met Gly Gly Leu Gly Lys Thr Thr Leu Ala
            180                 185                 190

Gln Met Val Phe Asn Asp Gln Arg Val Thr Glu His Phe His Ser Lys
        195                 200                 205

Ile Trp Ile Cys Val Ser Glu Asp Phe Asp Glu Lys Arg Leu Ile Lys
    210                 215                 220

Ala Ile Val Glu Ser Ile Glu Gly Arg Pro Leu Leu Gly Glu Met Asp
225                 230                 235                 240

Leu Ala Pro Leu Gln Lys Lys Leu Gln Glu Leu Leu Asn Gly Lys Arg
                245                 250                 255

Tyr Leu Leu Val Leu Asp Asp Val Trp Asn Glu Asp Gln Gln Lys Trp
            260                 265                 270

Ala Asn Leu Arg Ala Val Leu Lys Val Gly Ala Ser Gly Ala Ser Val
        275                 280                 285

Leu Thr Thr Thr Arg Leu Glu Lys Val Gly Ser Ile Met Gly Thr Leu
    290                 295                 300

Gln Pro Tyr Glu Leu Ser Asn Leu Ser Gln Glu Asp Cys Trp Leu Leu
305                 310                 315                 320

Phe Met Gln Arg Ala Phe Gly His Gln Glu Glu Ile Asn Pro Asn Leu
                325                 330                 335

Val Ala Ile Gly Lys Glu Ile Val Lys Lys Ser Gly Gly Val Pro Leu
            340                 345                 350

Ala Ala Lys Thr Leu Gly Gly Ile Leu Cys Phe Lys Arg Glu Glu Arg
        355                 360                 365

Ala Trp Glu His Val Arg Asp Ser Pro Ile Trp Asn Leu Pro Gln Asp
    370                 375                 380

Glu Ser Ser Ile Leu Pro Ala Leu Arg Leu Ser Tyr His Gln Leu Pro
385                 390                 395                 400

Leu Asp Leu Lys Gln Cys Phe Ala Tyr Cys Ala Val Phe Pro Lys Asp
                405                 410                 415

Ala Lys Met Glu Lys Glu Lys Leu Ile Ser Leu Trp Met Ala His Gly
            420                 425                 430

Phe Leu Leu Ser Lys Gly Asn Met Glu Leu Glu Asp Val Gly Asp Glu
        435                 440                 445

Val Trp Lys Glu Leu Tyr Leu Arg Ser Phe Phe Gln Glu Ile Glu Val
    450                 455                 460

Lys Asp Gly Lys Thr Tyr Phe Lys Met His Asp Leu Ile His Asp Leu
465                 470                 475                 480

Ala Thr Ser Leu Phe Ser Ala Asn Thr Ser Ser Ser Asn Ile Arg Glu
                485                 490                 495

Ile Asn Lys His Ser Tyr Thr His Met Met Ser Ile Gly Phe Ala Glu
            500                 505                 510

Val Val Phe Phe Tyr Thr Leu Pro Pro Leu Glu Lys Phe Ile Ser Leu
        515                 520                 525

Arg Val Leu Asn Leu Gly Asp Ser Thr Phe Asn Lys Leu Pro Ser Ser
    530                 535                 540

Ile Gly Asp Leu Val His Leu Arg Tyr Leu Asn Leu Tyr Gly Ser Gly
545                 550                 555                 560

Met Arg Ser Leu Pro Lys Gln Leu Cys Lys Leu Gln Asn Leu Gln Thr
                565                 570                 575

Leu Asp Leu Gln Tyr Cys Thr Lys Leu Cys Cys Leu Pro Lys Glu Thr
            580                 585                 590

Ser Lys Leu Gly Ser Leu Arg Asn Leu Leu Leu Asp Gly Ser Gln Ser
        595                 600                 605

Leu Thr Cys Met Pro Pro Arg Ile Gly Ser Leu Thr Cys Leu Lys Thr
    610                 615                 620

Leu Gly Gln Phe Val Val Gly Arg Lys Lys Gly Tyr Gln Leu Gly Glu
625                 630                 635                 640

Leu Gly Asn Leu Asn Leu Tyr Gly Ser Ile Lys Ile Ser His Leu Glu
                645                 650                 655

Arg Val Lys Asn Asp Lys Asp Ala Lys Glu Ala Asn Leu Ser Ala Lys
            660                 665                 670

Gly Asn Leu His Ser Leu Ser Met Ser Trp Asn Asn Phe Gly Pro His
        675                 680                 685

Ile Tyr Glu Ser Glu Glu Val Lys Val Leu Glu Ala Leu Lys Pro His
    690                 695                 700

Ser Asn Leu Thr Ser Leu Lys Ile Tyr Gly Phe Arg Gly Ile His Leu
705                 710                 715                 720

Pro Glu Trp Met Asn His Ser Val Leu Lys Asn Ile Val Ser Ile Leu
                725                 730                 735

Ile Ser Asn Phe Arg Asn Cys Ser Cys Leu Pro Pro Phe Gly Asp Leu
            740                 745                 750

Pro Cys Leu Glu Ser Leu Glu Leu His Trp Gly Ser Ala Asp Val Glu
        755                 760                 765

Tyr Val Glu Glu Val Asp Ile Asp Val His Ser Gly Phe Pro Thr Arg
    770                 775                 780

Ile Arg Phe Pro Ser Leu Arg Lys Leu Asp Ile Trp Asp Phe Gly Ser
785                 790                 795                 800

Leu Lys Gly Leu Leu Lys Lys Glu Gly Glu Glu Gln Phe Pro Val Leu
                805                 810                 815

Glu Glu Met Ile Ile His Glu Cys Pro Phe Leu Thr Leu Ser Ser Asn
            820                 825                 830

Leu Arg Ala Leu Thr Ser Leu Arg Ile Cys Tyr Asn Lys Val Ala Thr
        835                 840                 845

Ser Phe Pro Glu Glu Met Phe Lys Asn Leu Ala Asn Leu Lys Tyr Leu
    850                 855                 860

Thr Ile Ser Arg Cys Asn Asn Leu Lys Glu Leu Pro Thr Ser Leu Ala
865                 870                 875                 880

Ser Leu Asn Ala Leu Lys Ser Leu Lys Ile Gln Leu Cys Cys Ala Leu
                885                 890                 895

Glu Ser Leu Pro Glu Glu Gly Leu Glu Gly Leu Ser Ser Leu Thr Glu
            900                 905                 910

Leu Phe Val Glu His Cys Asn Met Leu Lys Cys Leu Pro Glu Gly Leu
        915                 920                 925

Gln His Leu Thr Thr Leu Thr Ser Leu Lys Ile Arg Gly Cys Pro Gln
    930                 935                 940

Leu Ile Lys Arg Cys Glu Lys Gly Ile Gly Glu Asp Trp His Lys Ile
945                 950                 955                 960

Ser His Ile Pro Asn Val Asn Ile Tyr Ile
                965                 970

<210>  20
<211>  8461
<212>  DNA
<213>  Solanum bulbocastanum

<220>
<221>  CDS
<222>  (2944)..(5487)

<400>  20
gatcaaaaac ggattcgggg agtgaaaagc ttaccgttag cctcaagaac gatggaaaac      60

tatcaagagt cgtctggggt tcgttcctta gctctaaaaa tcgaaaagtg cagaataaag     120

acgttttgag gcttatttac gcactacaaa aaaatcatat attgctgcaa aatttgttgt     180

agctaaatat gaaaatttcc atggctaaac ttcaatattt tcttccttag cattcgtagc     240

aagatatagc atggctaaaa gttagctaca aactttacat ggttcatggc tgagaactaa     300

tatctattgc tacaaaattt ttgagttgta gctgtactga taaaaagttt ttgccacaca     360

aaatatatat atatatatag caaataactt tattcttttg ccacgtcaaa aatgatttat     420

agttgtcttt ttagatgcag ccacaaagct attacattgt agcaacatat ttaatttggt     480

ttccatgtaa acagaaattt gtagctattt gtatagctta gtcacgtggc aatagtactc     540

gtatttagtt atgactatta aaattcatgg caataacatg aagtatttac cacaagcatt     600

ttcgaattat agctaaaatt catatcttta gccatgaata tatataattt tagctaaaaa     660

tccatttgct acgaaagtca aaatttatat acatgttttt ttacgtgata tatatagaaa     720

atttgttgca ttcaatatat aattccatta ttattagaga ttttttcgca agctttatta     780

gttccaagag ctacataatg tagctatgaa ttaattttta agatttcaaa acacttccta     840

aattacgtat aattacaatt tacaatttta aaaatagttc taattatata taactcatgt     900

aatacactat cttgtttgag tacaaagcag tatagtcttc tcttatttct ccttccacaa     960

cgttcaattg aatcttagct tgatttacac cgatcttgtc atttagatgc tttaactcca    1020

ttgatatacc tatcaaaatg atgaagtatt tcaacaccaa caatcattac caactaatta    1080

tagatgagag tgtgtgttat ataatatttc aagaagacaa caaatttatt tatatatata    1140

tcattagcta aaaaatttga aagatacaaa caaaaatatg caagaaaaaa aagtaaacaa    1200

atctataaac ttttatttct taaaataatg ttcacctcaa ttgtgacatt tttggcggtt    1260

tgcgaattat gactcctctc aacactattt aaattttaaa atcttcacaa tctgctcata    1320

aataagatgt aatatttttt aaaagatcta aaataatcta aacccaaaaa tctaagtcaa    1380

ataagtgaaa cataaattcg aaaattgtaa ataagaagat atgaacatgc cttgaatata    1440

aaaaaaccac aaaaagaaat tagcaaaaca ttctacattt tgaaatgcca aagtcttctc    1500

tctcaacatt tatctcttga gcaagaagat ttccatgtaa acttcatgtc ctttacttta    1560

agcattactt ccgatattgt tcttaccttt gtcaaggaac ctagtccatt gacctatggt 1620

ggacacagat aagctaacaa catttaatac cctaactacc attaccacaa caggtagagt 1680

acgctgaatt tttctaagtt gtgtcaccat ttaaaggaca aaaatgactc aagagtaaaa 1740

tcaatgaagc atttgctgca ggcctccaaa agttttatcg atatattttt ttttaataat 1800

ttgctcgttc ttccaaatca gttgaaactt gagttgttaa aattgatttg gtacgtctgg 1860

attttttttc aaataatacc gctccatcaa atttagatta atatgatgta atatgcacaa 1920

ttagaattgc ggacaattgt aaccaattta atgaattcaa aattatttca ttgtaacaag 1980

caaatagtaa aaaataaaat tattattatg aacaaataaa aagggacaag gcataagtac 2040

tctcctagac tatgactgaa atctcagaaa cacacataaa cttaactagg gtcctattac 2100

cccctaaact aatttaaaat ggaataaata caccacaaac ggtgacatga catagagagt 2160

gtacacactc tattgaaggc aattgattag tgcacaaatt ggacacatgt cattttttta 2220

ttgataactt tattattagt tagtacacat atttattgat aataaaaatt atatatatat 2280

aattattttt atttctttct ttgtaaaata tttattttaa tttcttttta ctttaaactt 2340

tttttatttt aatttattat gttttcactt ttgattattt aaaaaaaatt tatgtgtatt 2400

ttttaaaaaa gtaattttaa agtgtccttt aattttaatg ttttaacttt tttatgtttt 2460

atttgaattt cttcacttat tttaatatcc gttcaattta atgaatccta aattatttca 2520

ttgtaacaag taaatagaaa aaaataaaat aagtcttatg aagaaataaa aacaaatata 2580

ttttttccac aagttgtgta atgattgttg aagatgcttc cattttttaa atcttttctc 2640

aatatatatt ttccacaagt tgtgcaatga ttgctgaagt tgctttattg ttttaagact 2700

tttctctata tgtgtttttc cccaagttgt ataatggttg ttgaagatgc tttaattaaa 2760

aaaaaaaacc ttttgtttag tggaaaattt caaaaagctt tagtacatct ttgtcgtttt 2820

atccaatcgt aattctttat tcagaaacca catgtttttt ttctaatctt acttttatgt 2880

ctatcaccca ttttccaata tacagcctac tctttttttc aatcaaaact agtattccta 2940

aag atg gct gat gcc ttt cta tca ttt gca gtt caa aaa ttg ggt gat 2988
    Met Ala Asp Ala Phe Leu Ser Phe Ala Val Gln Lys Leu Gly Asp
    1               5                   10                  15

ttc cta ata cag aaa gtt tcc ctg cgt aaa agt ctc aga gat gaa att 3036
Phe Leu Ile Gln Lys Val Ser Leu Arg Lys Ser Leu Arg Asp Glu Ile
                20                  25                  30

aga tgg ctg ata aat gag cta ctc ttc ata cgg tct ttc ctc aga gat 3084
Arg Trp Leu Ile Asn Glu Leu Leu Phe Ile Arg Ser Phe Leu Arg Asp
            35                  40                  45

gca gaa caa aag cag tgc gga gat caa aga gtt caa caa tgg gtg ttt 3132
Ala Glu Gln Lys Gln Cys Gly Asp Gln Arg Val Gln Gln Trp Val Phe
        50                  55                  60

gag atc aac tct att gct aat gat gct gtt gct ata ctc gag act tat 3180
Glu Ile Asn Ser Ile Ala Asn Asp Ala Val Ala Ile Leu Glu Thr Tyr
    65                  70                  75

agc ttt gag gct ggt aaa ggt gct agt cgt ctc aag gct tgc act tgc 3228

Ser Phe Glu Ala Gly Lys Gly Ala Ser Arg Leu Lys Ala Cys Thr Cys
80                  85                  90                  95

ata tgt agg aag gag aag aaa ttc tac aat gtt gcc gag gag att caa      3276
Ile Cys Arg Lys Glu Lys Lys Phe Tyr Asn Val Ala Glu Glu Ile Gln
                100                 105                 110

tca ctc aag caa cga atc atg gat atc tct cgc aaa cga gag act tat      3324
Ser Leu Lys Gln Arg Ile Met Asp Ile Ser Arg Lys Arg Glu Thr Tyr
            115                 120                 125

ggt att aca aat atc aat tat aat tca gga gaa agg cca agt aat cag      3372
Gly Ile Thr Asn Ile Asn Tyr Asn Ser Gly Glu Arg Pro Ser Asn Gln
        130                 135                 140

gtt aca aca ttg agg aga act acc tca tat gta gat gaa cag gat tac      3420
Val Thr Thr Leu Arg Arg Thr Thr Ser Tyr Val Asp Glu Gln Asp Tyr
    145                 150                 155

att ttt gtt ggc ttt cag gat gtt gta caa aca ttg cta gct caa ctt      3468
Ile Phe Val Gly Phe Gln Asp Val Val Gln Thr Leu Leu Ala Gln Leu
160                 165                 170                 175

ctg aaa gca gag cct cgt cga agc gtc ctc tcc att tat gga atg ggg      3516
Leu Lys Ala Glu Pro Arg Arg Ser Val Leu Ser Ile Tyr Gly Met Gly
                180                 185                 190

ggt tta ggc aag acc act ctt gcc aga aaa ctt tac acc agt cct gat      3564
Gly Leu Gly Lys Thr Thr Leu Ala Arg Lys Leu Tyr Thr Ser Pro Asp
            195                 200                 205

ata ctc aat agc ttt cct aca cgc gct tgg ata tgt gtc tct caa gag      3612
Ile Leu Asn Ser Phe Pro Thr Arg Ala Trp Ile Cys Val Ser Gln Glu
        210                 215                 220

tac aac aca atg gat ctt ctt agg act atc ata aaa tcc atc caa ggc      3660
Tyr Asn Thr Met Asp Leu Leu Arg Thr Ile Ile Lys Ser Ile Gln Gly
    225                 230                 235

tgc gcc aag gaa act cta gat ttg ttg gaa aag atg gca gaa ata gat      3708
Cys Ala Lys Glu Thr Leu Asp Leu Leu Glu Lys Met Ala Glu Ile Asp
240                 245                 250                 255

cta gaa aat cac ctt cgt gat cta ttg aaa gaa tgc aaa tac ctt gtg      3756
Leu Glu Asn His Leu Arg Asp Leu Leu Lys Glu Cys Lys Tyr Leu Val
                260                 265                 270

gtg gtt gat gat gta tgg cag aga gaa gca tgg gag agt ttg aaa aga      3804
Val Val Asp Asp Val Trp Gln Arg Glu Ala Trp Glu Ser Leu Lys Arg
            275                 280                 285

gca ttc ccg gat ggc aag aat gga agc aga gtc att att acc acg cgc      3852
Ala Phe Pro Asp Gly Lys Asn Gly Ser Arg Val Ile Ile Thr Thr Arg
        290                 295                 300

aaa gag gat gtc gct gaa aga gta gac cac aga ggt ttt gtt cat aaa      3900
Lys Glu Asp Val Ala Glu Arg Val Asp His Arg Gly Phe Val His Lys
    305                 310                 315

ctt cgt ttc cta agt caa gaa gaa agt tgg gat ctc ttt cgt agg aaa      3948
Leu Arg Phe Leu Ser Gln Glu Glu Ser Trp Asp Leu Phe Arg Arg Lys
320                 325                 330                 335

cta ctt gat gtt cga gca atg gtt cca gaa atg gaa agt tta gct aag      3996
Leu Leu Asp Val Arg Ala Met Val Pro Glu Met Glu Ser Leu Ala Lys
                340                 345                 350

gat atg gtg gaa aag tgt aga ggc tta cct ctt gca att gtt gta ttg      4044

Asp Met Val Glu Lys Cys Arg Gly Leu Pro Leu Ala Ile Val Val Leu
        355                 360                 365

agc gga cta ctt tcg cat aaa aag ggg cta aac caa tgg caa aag gtg      4092
Ser Gly Leu Leu Ser His Lys Lys Gly Leu Asn Gln Trp Gln Lys Val
        370                 375                 380

aaa gat cac ctt tgg aag aac att aaa gaa gat aaa tct att gaa atc      4140
Lys Asp His Leu Trp Lys Asn Ile Lys Glu Asp Lys Ser Ile Glu Ile
    385                 390                 395

tct aac ata cta tcc tta agc tac aat gat ttg tca act gcg ctc aag      4188
Ser Asn Ile Leu Ser Leu Ser Tyr Asn Asp Leu Ser Thr Ala Leu Lys
400                 405                 410                 415

cag tgt ttt ctc tac ttt ggt att ttt cca gaa gat caa gtg gta aag      4236
Gln Cys Phe Leu Tyr Phe Gly Ile Phe Pro Glu Asp Gln Val Val Lys
                420                 425                 430

gct gat gac ata ata cgg ttg tgg atg gcg gag ggt ttc ata ccc aga      4284
Ala Asp Asp Ile Ile Arg Leu Trp Met Ala Glu Gly Phe Ile Pro Arg
        435                 440                 445

gga gaa gaa aga atg gag gat gtg gct gac ggc ttc ttg aat gaa ctg      4332
Gly Glu Glu Arg Met Glu Asp Val Ala Asp Gly Phe Leu Asn Glu Leu
        450                 455                 460

ata aga cga agc ttg gtt caa gta gct aaa aca ttt tgg gaa aaa gtt      4380
Ile Arg Arg Ser Leu Val Gln Val Ala Lys Thr Phe Trp Glu Lys Val
    465                 470                 475

act gac tgt agg gtt cat gat tta ctt cgt gat ctt gcg ata caa aag      4428
Thr Asp Cys Arg Val His Asp Leu Leu Arg Asp Leu Ala Ile Gln Lys
480                 485                 490                 495

gca ttg gag gta aac ttc ttt gac gtt tat ggt cca aga agc cac tcc      4476
Ala Leu Glu Val Asn Phe Phe Asp Val Tyr Gly Pro Arg Ser His Ser
                500                 505                 510

ata tcc tct tta tgt atc aga cat ggc att cat agt gaa gga gaa agg      4524
Ile Ser Ser Leu Cys Ile Arg His Gly Ile His Ser Glu Gly Glu Arg
        515                 520                 525

tac ctc tca tca ctt gat ctt tct aac ttg aag ttg agg tca att atg      4572
Tyr Leu Ser Ser Leu Asp Leu Ser Asn Leu Lys Leu Arg Ser Ile Met
        530                 535                 540

ttc ttc gat cca gat ttt cgt aag atg agt cat ata aac ctc agg agt      4620
Phe Phe Asp Pro Asp Phe Arg Lys Met Ser His Ile Asn Leu Arg Ser
    545                 550                 555

gag ttc caa cat ctg tat gtg ttg tac ttg gat acg aat ttt ggg tat      4668
Glu Phe Gln His Leu Tyr Val Leu Tyr Leu Asp Thr Asn Phe Gly Tyr
560                 565                 570                 575

gtg tct atg gta cct gat gcc ata gga agt ttg tac cac ctc aag ttg      4716
Val Ser Met Val Pro Asp Ala Ile Gly Ser Leu Tyr His Leu Lys Leu
                580                 585                 590

tta aga ttg aga ggt atc cat gat att ccg tct tcc att ggc aac ctc      4764
Leu Arg Leu Arg Gly Ile His Asp Ile Pro Ser Ser Ile Gly Asn Leu
        595                 600                 605

aag aat tta caa aca ctt gtc gtt gta aat ggt tac aca ttt ttt tgc      4812
Lys Asn Leu Gln Thr Leu Val Val Val Asn Gly Tyr Thr Phe Phe Cys
        610                 615                 620

caa cta ccc tgc aag aca gct gac cta ata aat cta aga cat tta gtt      4860

Gln Leu Pro Cys Lys Thr Ala Asp Leu Ile Asn Leu Arg His Leu Val
    625                 630                 635

gtt caa tat tca gag cct tta aaa tgt ata aac aaa ctc act agt ctt     4908
Val Gln Tyr Ser Glu Pro Leu Lys Cys Ile Asn Lys Leu Thr Ser Leu
640                 645                 650                 655

caa gtt ctt gat ggt gtt gct tgt gat cag tgg aaa gat gtt gac cct     4956
Gln Val Leu Asp Gly Val Ala Cys Asp Gln Trp Lys Asp Val Asp Pro
                660                 665                 670

gtt gat tta gtc aat ctt cga gaa tta agc atg gat cgt atc agg agc     5004
Val Asp Leu Val Asn Leu Arg Glu Leu Ser Met Asp Arg Ile Arg Ser
            675                 680                 685

tct tac tcc cta aac aac att agc agc ttg aaa aac ctt agc act ctc     5052
Ser Tyr Ser Leu Asn Asn Ile Ser Ser Leu Lys Asn Leu Ser Thr Leu
        690                 695                 700

aaa ttg att tgt gga gaa cgt caa tca ttt gca tcc ctt gaa ttt gtt     5100
Lys Leu Ile Cys Gly Glu Arg Gln Ser Phe Ala Ser Leu Glu Phe Val
    705                 710                 715

aat tgt tgt gaa aag ctc cag aaa ttg tgg tta caa ggg aga ata gag     5148
Asn Cys Cys Glu Lys Leu Gln Lys Leu Trp Leu Gln Gly Arg Ile Glu
720                 725                 730                 735

gaa ctg cct cat ctg ttt tca aac tcc atc aca atg atg gtt ctg agt     5196
Glu Leu Pro His Leu Phe Ser Asn Ser Ile Thr Met Met Val Leu Ser
                740                 745                 750

ttc tca gaa ctg aca gaa gat ccg atg cct att ttg gga agg ttt cca     5244
Phe Ser Glu Leu Thr Glu Asp Pro Met Pro Ile Leu Gly Arg Phe Pro
            755                 760                 765

aac cta agg aat ctc aaa tta gat gga gct tat gaa gga aaa gaa ata     5292
Asn Leu Arg Asn Leu Lys Leu Asp Gly Ala Tyr Glu Gly Lys Glu Ile
        770                 775                 780

atg tgc agt gat aac agc ttc agt caa cta gag ttc ctt cat ctt cgt     5340
Met Cys Ser Asp Asn Ser Phe Ser Gln Leu Glu Phe Leu His Leu Arg
    785                 790                 795

gat ctt tgg aag cta gaa aga tgg gat tta ggc aca agt gcc atg cct     5388
Asp Leu Trp Lys Leu Glu Arg Trp Asp Leu Gly Thr Ser Ala Met Pro
800                 805                 810                 815

ctg att aaa ggt ctt ggt atc cat aac tgt cca aat tta aag gag att     5436
Leu Ile Lys Gly Leu Gly Ile His Asn Cys Pro Asn Leu Lys Glu Ile
                820                 825                 830

cct gag aga atg aaa gac atg gag ctg ttg aag cgg aat tat atg ttg     5484
Pro Glu Arg Met Lys Asp Met Glu Leu Leu Lys Arg Asn Tyr Met Leu
            835                 840                 845

tga agcttttctg ccaagcacat tggttattaa ttgagtggtt ttagtgttga          5537

tttcttatta ttgttttaag ctttttgagt gtgtaattgg tttgaacatt attgttttaa   5597

ttaattggtc tactgtatgt tctcatgctt atccacattt aagacaatgc tttatatgtt   5657

aaaatgaaat taaaaatact agtatatggt actctctctt gtccacaatt tcgtatattt   5717

tttgttcctc ttcataaaaa aaatggtaaa aaataccatt aaactatgtg ataggaacaa   5777

aaatgtcttc tattataatt taacttaaaa atgtctttac tgtcagtacc ttagttcaaa   5837

attgccctcg agtccgtagt tacaaaatgt cctttttcga ataaatatat atattttttt   5897

aaacacatct tcttcctaat taaaatatta ttaaagaaga ctattcttgt tttctttttt 5957
ctaaaaatca ctttaacaaa taaaaatgta ggaaatattt tgttttcttc ttaatctcac 6017
tttatcaatt aaaatagaat aactccatgt accctttcga catataatat atgtcatata 6077
tatatataag atcatagtat atgcatcatt aatttatatt tatataacga taaaaaataa 6137
tgataataaa ataagaaata tttttatttt ttattttctt ctgaattgaa gtaatataaa 6197
catttgctaa ttttaaaaaa aaataataca aaaataatgt gttaaaaaga aaataataat 6257
atatttattt ggaaaatgag tatttttgat ccattaaata acagtaagtg tatttttaga 6317
ccaaagtatt gacaacaagg gtatttttgg atcaaacgac aaacggaggg tacttttgct 6377
cctttcgcat aatttaaggg tatttttaaa ccaaaatatt gacggtaaag gcatttttga 6437
gtcaaattat gaacgaaaga catttttatt tctttcacat agtttaagga catttttgac 6497
ccatttccct cctttatata aataatattt atgttaaatc aacagagaag aagctgtcaa 6557
ttgaagacat tcactttcat caacttggct tctccaagca tcaatcaact tggattattt 6617
caacattctg ttttttcaat gtttaatttc tttctatttt tggaaacatg tgttggaaga 6677
gaaccttttt tctggatttt gtgatgacct aattaacgaa acaaagttaa aaatgttctt 6737
aaattatgta aaatgaataa aaatatcctc agttaatagt ttgatccaaa aatattgttg 6797
tctctaataa ttgatctaaa aatgatatta ttgttactta ataagtgaaa accgtctttt 6857
tttcaattaa atatattatt tttctttttt taaaacacgc tcttttccta ataatgattt 6917
tttttcatta aaaaatattc atcctacttc aatttataaa aaatattact aataaataaa 6977
aatgtttttt atattattag aaagtttttt atcgttattt aaatgaaaat taatgacggg 7037
gttactatga ggacatataa tggaagaagt tgagaactcg cttagtgtga agcgagaata 7097
actaaaaaaa aaaaaaaaac ttacaaactc gcttggtgcg gagcgatttt tgggggaaaa 7157
tagagagcaa atcgctcata ggtagcgaga aaaaaaagat aaaataaaag agaaaatcgc 7217
tcgtaagtca acaagatgat caattttttt atgccgttaa cgatagttat agcacaaaca 7277
tatctcgctc cttctctagc aaagtgtccc ttttgattaa accaaaaatt gaaagaccct 7337
ttatgtattt taaagaaaaa aagtgatgtt ttttaacttg aattcgaaat ttaatcatcc 7397
caatataatt cataaacgaa tttttacatc aattttaaaa taaagaataa aaaaaagaaa 7457
gataatatat actagcaggg aactacatgt gattactaca aaagataaat tcaatttcag 7517
gtggtatttg gatttgaatt gtcttacctt gctatcataa cattattttt gtttttatcc 7577
attaaaaaaa tgatgcattt atatatttat tactagtaaa gtaatatctt taatgtgtca 7637
acacataagt atccctcaat tagtaaaatg ttaggacttt tttcatgtga gaaacccaac 7697
ctcattgaaa aaggaaatta atacatttta actcaacttt taattaatta atgtcaagtt 7757
tgataaaaat aaataaaaaa acaatcgtag acaatctcta attattagaa ttttacaata 7817
tgcatattta atgggttata taaattttga gttggccttc tttttttctt cttgtgattc 7877
taagtcctcc actttatttt tatttttata tttataatta aatatttttt actcgattca 7937

cagaccgagt tggaccagtc caatcttgat taagcctcac gagttgacga gcttatttag 7997

gcttggctaa ataatttcgt tcttaaatga acttttaatt ttttttgagc tcaatcctat 8057

caaatcgcag attaggttgg atttgggtga aacaatggac caaagtccaa actaacagct 8117

ccaaaatcta cgaggtttag aaatagaaag tcttcttata tgttatgtat atctaacaaa 8177

ttatatgtta tgtatgatat tgtataaata gttatttaat gtatcaatat tgtataataa 8237

catatagtta tgtatttata atgtataact atatatgata ttgtataaat agttatttaa 8297

tgtatcgata ttgtatagta gcatatagtt atgtatttat aatgtatgta taactatgta 8357

tgatattgta taaatagatt ttggacttgg gaagtctgca gcaagcaaag gaagaggtcc 8417

aggtagcaac acttttatct taatgaatac accaaatgat gatc 8461

<210> 21
<211> 847
<212> PRT
<213> Solanum bulbocastanum

<400> 21

Met Ala Asp Ala Phe Leu Ser Phe Ala Val Gln Lys Leu Gly Asp Phe
1               5                   10                  15

Leu Ile Gln Lys Val Ser Leu Arg Lys Ser Leu Arg Asp Glu Ile Arg
            20                  25                  30

Trp Leu Ile Asn Glu Leu Leu Phe Ile Arg Ser Phe Leu Arg Asp Ala
        35                  40                  45

Glu Gln Lys Gln Cys Gly Asp Gln Arg Val Gln Gln Trp Val Phe Glu
    50                  55                  60

Ile Asn Ser Ile Ala Asn Asp Ala Val Ala Ile Leu Glu Thr Tyr Ser
65                  70                  75                  80

Phe Glu Ala Gly Lys Gly Ala Ser Arg Leu Lys Ala Cys Thr Cys Ile
                85                  90                  95

Cys Arg Lys Glu Lys Lys Phe Tyr Asn Val Ala Glu Glu Ile Gln Ser
            100                 105                 110

Leu Lys Gln Arg Ile Met Asp Ile Ser Arg Lys Arg Glu Thr Tyr Gly
        115                 120                 125

Ile Thr Asn Ile Asn Tyr Asn Ser Gly Glu Arg Pro Ser Asn Gln Val
    130                 135                 140

Thr Thr Leu Arg Arg Thr Thr Ser Tyr Val Asp Glu Gln Asp Tyr Ile
145                 150                 155                 160

Phe Val Gly Phe Gln Asp Val Val Gln Thr Leu Leu Ala Gln Leu Leu

                165                 170                 175

Lys Ala Glu Pro Arg Arg Ser Val Leu Ser Ile Tyr Gly Met Gly Gly
            180                 185                 190

Leu Gly Lys Thr Thr Leu Ala Arg Lys Leu Tyr Thr Ser Pro Asp Ile
        195                 200                 205

Leu Asn Ser Phe Pro Thr Arg Ala Trp Ile Cys Val Ser Gln Glu Tyr
    210                 215                 220

Asn Thr Met Asp Leu Leu Arg Thr Ile Ile Lys Ser Ile Gln Gly Cys
225                 230                 235                 240

Ala Lys Glu Thr Leu Asp Leu Leu Glu Lys Met Ala Glu Ile Asp Leu
                245                 250                 255

Glu Asn His Leu Arg Asp Leu Leu Lys Glu Cys Lys Tyr Leu Val Val
            260                 265                 270

Val Asp Asp Val Trp Gln Arg Glu Ala Trp Glu Ser Leu Lys Arg Ala
        275                 280                 285

Phe Pro Asp Gly Lys Asn Gly Ser Arg Val Ile Ile Thr Thr Arg Lys
    290                 295                 300

Glu Asp Val Ala Glu Arg Val Asp His Arg Gly Phe Val His Lys Leu
305                 310                 315                 320

Arg Phe Leu Ser Gln Glu Glu Ser Trp Asp Leu Phe Arg Arg Lys Leu
                325                 330                 335

Leu Asp Val Arg Ala Met Val Pro Glu Met Glu Ser Leu Ala Lys Asp
            340                 345                 350

Met Val Glu Lys Cys Arg Gly Leu Pro Leu Ala Ile Val Val Leu Ser
        355                 360                 365

Gly Leu Leu Ser His Lys Lys Gly Leu Asn Gln Trp Gln Lys Val Lys
    370                 375                 380

Asp His Leu Trp Lys Asn Ile Lys Glu Asp Lys Ser Ile Glu Ile Ser
385                 390                 395                 400

Asn Ile Leu Ser Leu Ser Tyr Asn Asp Leu Ser Thr Ala Leu Lys Gln
                405                 410                 415

Cys Phe Leu Tyr Phe Gly Ile Phe Pro Glu Asp Gln Val Val Lys Ala
            420                 425                 430

Asp Asp Ile Ile Arg Leu Trp Met Ala Glu Gly Phe Ile Pro Arg Gly

        435                 440                 445

Glu Glu Arg Met Glu Asp Val Ala Asp Gly Phe Leu Asn Glu Leu Ile
    450                 455                 460

Arg Arg Ser Leu Val Gln Val Ala Lys Thr Phe Trp Glu Lys Val Thr
465                 470                 475                 480

Asp Cys Arg Val His Asp Leu Leu Arg Asp Leu Ala Ile Gln Lys Ala
                485                 490                 495

Leu Glu Val Asn Phe Phe Asp Val Tyr Gly Pro Arg Ser His Ser Ile
            500                 505                 510

Ser Ser Leu Cys Ile Arg His Gly Ile His Ser Glu Gly Glu Arg Tyr
        515                 520                 525

Leu Ser Ser Leu Asp Leu Ser Asn Leu Lys Leu Arg Ser Ile Met Phe
    530                 535                 540

Phe Asp Pro Asp Phe Arg Lys Met Ser His Ile Asn Leu Arg Ser Glu
545                 550                 555                 560

Phe Gln His Leu Tyr Val Leu Tyr Leu Asp Thr Asn Phe Gly Tyr Val
                565                 570                 575

Ser Met Val Pro Asp Ala Ile Gly Ser Leu Tyr His Leu Lys Leu Leu
            580                 585                 590

Arg Leu Arg Gly Ile His Asp Ile Pro Ser Ser Ile Gly Asn Leu Lys
        595                 600                 605

Asn Leu Gln Thr Leu Val Val Val Asn Gly Tyr Thr Phe Phe Cys Gln
    610                 615                 620

Leu Pro Cys Lys Thr Ala Asp Leu Ile Asn Leu Arg His Leu Val Val
625                 630                 635                 640

Gln Tyr Ser Glu Pro Leu Lys Cys Ile Asn Lys Leu Thr Ser Leu Gln
                645                 650                 655

Val Leu Asp Gly Val Ala Cys Asp Gln Trp Lys Asp Val Asp Pro Val
            660                 665                 670

Asp Leu Val Asn Leu Arg Glu Leu Ser Met Asp Arg Ile Arg Ser Ser
        675                 680                 685

Tyr Ser Leu Asn Asn Ile Ser Ser Leu Lys Asn Leu Ser Thr Leu Lys
    690                 695                 700

Leu Ile Cys Gly Glu Arg Gln Ser Phe Ala Ser Leu Glu Phe Val Asn

705                 710                 715                 720

Cys Cys Glu Lys Leu Gln Lys Leu Trp Leu Gln Gly Arg Ile Glu Glu
                725                 730                 735

Leu Pro His Leu Phe Ser Asn Ser Ile Thr Met Met Val Leu Ser Phe
            740                 745                 750

Ser Glu Leu Thr Glu Asp Pro Met Pro Ile Leu Gly Arg Phe Pro Asn
        755                 760                 765

Leu Arg Asn Leu Lys Leu Asp Gly Ala Tyr Glu Gly Lys Glu Ile Met
    770                 775                 780

Cys Ser Asp Asn Ser Phe Ser Gln Leu Glu Phe Leu His Leu Arg Asp
785                 790                 795                 800

Leu Trp Lys Leu Glu Arg Trp Asp Leu Gly Thr Ser Ala Met Pro Leu
                805                 810                 815

Ile Lys Gly Leu Gly Ile His Asn Cys Pro Asn Leu Lys Glu Ile Pro
            820                 825                 830

Glu Arg Met Lys Asp Met Glu Leu Leu Lys Arg Asn Tyr Met Leu
        835                 840                 845